# EUROPEAN PATENT APPLICATION

(11) **EP 2 206 518 A1**
(43) Date of publication of application: **14.07.2010**
(21) Application number: 08842089.8
(22) Date of filing: 23.10.2008
(51) Int. Cl.: A61K 45/08, A61K 31/365, A61K 31/436, A61K 31/5377, A61K 38/00, A61K 47/26, A61K 47/34, A61P 43/00

(54) **PHARMACEUTICAL COMPOSITION CONTAINING LIPOPHILIC IL-2 PRODUCTION INHIBITOR**

(30) Priority: 25.10.2007 US 982664 P; 05.02.2008 US 26244 P; 15.02.2008 US 29291 P
(71) Applicant: Astellas Pharma Inc., Tokyo 103-8411 (JP)
(72) Inventor: YOSHIDA, Takayuki, Tokyo 103-8411 (JP); NAKANISHI, Kiyo, Tokyo 103-8411 (JP); MAEDA, Atsushi, Tokyo 103-8411 (JP); SAKO, Kazuhiro, Tokyo 103-8411 (JP); KASASHIMA, Yuki, Tokyo 103-8411 (JP); KONDO, Hiromu, Tokyo 103-8411 (JP); YOSHIOKA, Tatsunobu, Tokyo 103-8411 (JP); TSUTSUI, Yuuki, Tokyo 103-8411 (JP)
(74) Representative: Gille Hrabal Struck Neidlein Prop Roos
(86) International application number: PCT/JP2008/069250
(87) International publication number: WO 2009/054463

(57) **Abstract**

A pharmaceutical composition comprising a lipophilic substance which inhibits IL-2 production, and a base capable of inhibiting blood exposure of the substance and delivering the substance to lymph following oral administration, is disclosed. The pharmaceutical composition can inhibit blood exposure of the substance to reduce its adverse effects, and can develop desired pharmacological effects.

## Description

### TECHNICAL FIELD

The present invention relates to a pharmaceutical composition comprising a lipophilic substance which inhibits IL-2 production, and a carrier capable of inhibiting blood exposure of the substance when orally administered and of delivering the substance to lymph in an amount necessary for developing its effects. More particularly, the present invention relates to a pharmaceutical composition which inhibits the transfer of a drug to blood and accomplishes an efficient delivery of the drug to lymph.
Further, the present invention relates to a method of inhibiting blood exposure of a lipophilic substance which inhibits IL-2 production, or a method of delivering the substance to lymph, by orally administrating the pharmaceutical composition comprising the substance and a carrier capable of inhibiting the transfer of the substance to blood and of delivering the substance to lymph.
Furthermore, the present invention relates to a use of specific fats and oils or specific surfactants for inhibiting the transfer of a lipophilic substance which inhibits IL-2 production to blood following oral administration, and delivering the substance to lymph.

### BACKGROUND ART

With respect to lipophilic substances which inhibit IL-2 production, tacrolimus, cyclosporin, rapamycin, and the like are known to be substances which inhibit lymphocyte interleukin-2 (IL-2) production (patent reference 1).
Furthermore, mycophenolic acid and mycophenolate mofetil inhibit proliferation of lymphocytes thereby inhibiting IL-2 production, and thus, are known as substances which inhibit IL-2 production (patent reference 2 and non-patent reference 1).

The lipophilic substances which inhibit IL-2 production have a low water-solubility as physicochemical properties, and a low bioavailability when orally administered as a solid or an aqueous suspension. Due to these properties, for example, the current tacrolimus product (product name: Prograf) is provided in the medical field, on the basis of an invention relating to a solid dispersion composition which is composed of tacrolimus and a polymer such as hydroxypropyl methylcellulose (HPMC) and has an enhanced bioavailability (patent reference 3).

However, it has been reported as warning that absorption of a lipophilic substance which inhibits IL-2 production is variable and dependent on patients after oral administration, and thus, it is preferred to measure its blood concentrations in accordance with the condition of a patient (in particular, to frequently measure its blood concentrations immediately after transplantation or the beginning of administration) and control the dose on the basis of the blood trough concentrations, to avoid adverse effects at high blood concentrations and avoid rejection and the onset of graft versus host disease at low blood concentrations (nonpatent reference 2, see <USAGE AND DOSE>).

To improve oral absorption and a variable range of absorption, an oral pharmaceutical composition containing an amorphous lipophilic substance which inhibits IL-2 production and one or more surfactants has been reported (patent reference 4). Further, to avoid a decreased bioavailability based on CYP metabolism in the gastrointestinal tract, a controlled-release oral formulation in which a solid dispersion composition of a lipophilic substance which inhibits IL-2 production is coated with an enteric macromolecule has been reported (patent reference 5). Furthermore, an invention relating to a non-aqueous formulation suitable for intramuscular injection, in which a lipophilic substance which inhibits IL-2 production is dissolved together with an oil in a nonaqueous solvent, and an invention relating to a non-aqueous formulation in which an emulsifier may be further added if desired, have been reported (patent reference 6).

With respect to lipophilic substances which inhibit IL-2 production, an increased incidence of adverse events, such as abnormal renal function, or abnormal pancreatic function including hyperglycemia, diabetes mellitus, and the like, has been reported (nonpatent reference 2, see <INDICATIONS AND USAGE>).
Further, it has been reported that there is a relationship between blood exposure of a lipophilic substance which inhibits IL-2 production following administration and its pharmacological effects, but continuous high blood concentrations often cause nephropathy (nonpatent reference 2, see <INDICATIONS CONCERNING USAGE AND DOSE>).

Under these circumstances, with respect to lipophilic substances which inhibit IL-2 production of which a remarkable immunosuppressive effect has been clinically identified, a pharmaceutical composition containing such a substance which can alleviate adverse effects by inhibiting blood exposure thereof and shows its pharmacological effect, an immunosuppressive effect, has been desired.

Rejection following transplantation is based on lymphocytes, and the action of immunosuppressants on lymphocytes is known as its pharmacological mechanism of action. Lymphatic delivery technologies enable an immunosuppressant orally administered to directly act on lymphocytes located in the lymphatic system, not by delivering it into blood vessels via gastrointestinal mucosa, but by directly delivering it into lymphatic vessels.

However, since a remarkably increased concentration of immunosuppressant in lymph enhances the risk of occurrence of lymphoma, it is preferable to efficiently deliver an immunosuppressant to lymph in an amount necessary for developing its effects.

As such lymphatic delivery technologies, a formulation for lymphatic delivery in which a surfactant, such as glycerin fatty acid ester, sugar fatty acid ester, sorbitan fatty acid ester, or the like, is used to disperse cyclosporins into water has been reported, and it has been reported that a medium or higher fatty acid, such as myristic acid, palmitic acid, stearic acid, oleic acid, or the like, may be added to the formulation, if desired (patent reference 7 and nonpatent reference 3). In this technology, the blood concentration of cyclosporin is not altered. Further, the lymph concentration thereof is remarkably increased to enhance the risk of occurrence of lymphoma.

To accomplish stable and high absorption and the enhancement of bioavailability, an emulsion formulation having a diameter of 200 nm or more, containing a compound such as tacrolimus (FK506) or a rapamycin class; a lipophilic phase such as medium chain triglycerides (TG), mixed mono-, di-, and tri-glycerides (conveniently, for example, almond oil, peanut oil, peach oil, palm oil, corn oil, sunflower oil, safflower oil, or the like), or a transesterified ethoxylated vegetable oil; a hydrophilic phase; and a surfactant has been reported (patent reference 8). However, an oral pharmaceutical composition having enhanced bioavailability (i.e., increased blood concentration of tacrolimus) is exemplified, but a technique for inhibiting blood exposure of a lipophilic substance which inhibits IL-2 production and delivering the substance to lymph in an amount necessary for developing its effects is not disclosed in this reference.

[patent reference 1] Japanese Unexamined Patent Publication (Kokai) No. 61-148181 (corresponding to European Patent Publication No. 0323042)
[patent reference 2] United States Patent No. 4786637, Japanese Patent No. 3844491
[patent reference 3] Japanese Unexamined Patent Publication (Kokai) No. 62-277321
[patent reference 4] International Publication No. 98/24418
[patent reference 5] International Publication No. 99/49863
[patent reference 6] Japanese Unexamined Patent Publication (Kokai) No. 4-182429
[patent reference 7] Japanese Unexamined Patent Publication (Kokai) No. 61-280435
[patent reference 8] Japanese Unexamined Patent Publication (Kokai) No. 7-138161 (corresponding to United States Patents Nos. 5932243, 6565859, and 7025975, and United States Patent Application No. 2003/0166517A1)
[non-patent reference 1] Transplant 64, 1695, 1997
[non-patent reference 2] Document attached to Prograf capsules 0.5 mg and 1 mg
[non-patent reference 3] Pharma. Res., 3(1), 48-51, 1986

### DISCLOSURE OF THE INVENTION

### PROBLEMS TO BE SOLVED BY THE INVENTION

The present invention is to provide a pharmaceutical composition comprising a lipophilic substance which inhibits IL-2 production, of which a remarkable immunosuppressive effect has been clinically identified. The pharmaceutical composition of the present invention can alleviate adverse effects by inhibiting blood exposure of the substance, and can develop its pharmacological effect, an immunosuppressive effect.
Further, the present invention is to provide a method of alleviating adverse effects of a lipophilic substance which inhibits IL-2 production, by orally administering the pharmaceutical composition containing a carrier capable of inhibiting a transfer of the substance to blood and of delivering the substance to lymph, to thereby inhibit blood exposure of the substance and develop its pharmacological effect, an immunosuppressive effect.
Furthermore, the present invention is to provide a use of a carrier for inhibiting blood exposure of a lipophilic substance which inhibits IL-2 production and delivering the substance to lymph.

### MEANS FOR SOLVING THE PROBLEMS

The present inventors focused on a main effect of lipophilic substances which inhibit IL-2 production, an effect of inhibiting IL-2 production, and an index of adverse effects of the substances, blood exposure, and found bases which can inhibit blood exposure of the substance and can develop its pharmacological effect, an immunosuppressive effect. Further, on the basis of these findings, a method of alleviating adverse effects of lipophilic substances which inhibit IL-2 production, and a use of a carrier for lymphatic delivery were examined to complete the present invention.

The present invention relates to
[1] a pharmaceutical composition comprising a lipophilic substance which inhibits IL-2 production, and a carrier capable of inhibiting blood exposure of the substance and delivering the substance to lymph;
[2] the pharmaceutical composition of [1], wherein the lipophilic substance which inhibits IL-2 production has a LogP value of 2 or more and an IC₅₀ (a 50% inhibitory drug concentration in a human mixed lymphocyte reaction) of 1 µmol/L or less;
[3] the pharmaceutical composition of [1] or [2], wherein the lipophilic substance which inhibits IL-2 production is one or two or more substances selected from the group consisting of tacrolimus, cyclosporin, rapamycin, mycophenolic acid, and mycophenolate mofetil;
[4] the pharmaceutical composition of [1] to [3], wherein the carrier capable of inhibiting exposure to the substance in blood and delivering the substance to lymph is one or two or more substances selected from the group consisting of (i) a mono-, di-, or tri-glyceride, or a mixture thereof, (ii) a fatty acid having 12 to 18 carbon atoms, or a mixture thereof, (iii) a surfactant having an HLB value of 10 to 18 and containing polyethylene glycol having a weight average molecular weight of 200 to 5000 in its molecular structure, and (iv) a higher alcohol having 12 to 18 carbon atoms;
[5] the pharmaceutical composition of [4], wherein (i) is one or two or more substances selected from the group consisting of rapeseed oil, castor oil, sunflower oil, cacao butter, medium chain triglycerides, monoolein, diolein, triolein, monoglyceride stearate, diglyceride stearate, triglyceride stearate, monoglyceride palmitate, diglyceride palmitate, triglyceride palmitate, monoglyceride myristate, diglyceride myristate, triglyceride myristate, monoglyceride arachidate, diglyceride arachidate, triglyceride arachidate, monoglyceride behenate, diglyceride behenate, and triglyceride behenate;
[6] the pharmaceutical composition of [4], wherein (ii) is one or two or more substances selected from the group consisting of linoleic acid, stearic acid, oleic acid, lauric acid, and palmitic acid;
[7] the pharmaceutical composition of [4], wherein (iii) is one or two or more substances selected from the group consisting of polyoxyethylene polyoxypropylene glycol, polyoxyethylene fatty acid monoester, saturated polyglycosylated glyceride, D-α-tocopheryl polyethylene glycol 1000 succinate, and polyoxyethylene hydrogenated castor oil;
[8] the pharmaceutical composition of [4], wherein (iv) is one or two or more substances selected from the group consisting of stearyl alcohol, myristyl alcohol, and cetyl alcohol;
[9] the pharmaceutical composition of [1] to [8], wherein the content of the carrier is 0.1 to 20,000 parts by weight with respect to 1 part by weight of the lipophilic substance which inhibits IL-2 production;
[10] the pharmaceutical composition of [1], which is for oral administration;
[11] the pharmaceutical composition of [1], which is for lymphatic delivery;
[12] a method of delivering a lipophilic substance which inhibits IL-2 production to lymph, comprising administering to a subject, a carrier capable of inhibiting blood exposure of the substance and delivering the substance to lymph;
[13] the method of [12], wherein a period of time in which C_{lymph}/IC₅₀ is higher than 30 lasts for 1 hour or more in the lymphatic delivery;
[14] method of [12] or [13], wherein a period of time in which C_{lymph}/IC₅₀ is higher than 30 lasts for 4 hours or less in the lymphatic delivery;
[15] the method of [12], wherein blood AUC_{blood} or plasma AUCₚₗₐₛₘₐ as an index of blood exposure of a lipophilic substance which inhibits IL-2 production is 0% to 80% in comparison with that of a control composition;
[16] the method of [12], wherein the lipophilic substance which inhibits IL-2 production has a LogP value of 2 or more and an IC₅₀ (a 50% inhibitory drug concentration in a human mixed lymphocyte reaction) of 1 µmol/L or less;
[17] the method of [12], wherein the lipophilic substance which inhibits IL-2 production is one or two or more substances selected from the group consisting of tacrolimus, cyclosporin, rapamycin, mycophenolic acid, and mycophenolate mofetil;
[18] the method of [12], wherein the carrier is orally administered;
[19] the method of [12], wherein the pharmaceutical composition according to any one of claims 1 to 11 is administered;
[20] use of one or two or more substances selected from the group consisting of (i) a mono-, di-, or tri-glyceride, or a mixture thereof, (ii) a fatty acid having 12 to 18 carbon atoms, or a mixture thereof, (iii) a surfactant having an HLB value of 10 to 18 and containing polyethylene glycol having a weight average molecular weight of 200 to 5000 in its molecular structure, and (iv) a higher alcohol having 12 to 18 carbon atoms, as a carrier capable of inhibiting blood exposure of a lipophilic substance which inhibits IL-2 production and delivering the substance to lymph;
[21] use of [20], wherein (i) is one or two or more substances selected from the group consisting of rapeseed oil, castor oil, sunflower oil, cacao butter, medium chain triglycerides, monoolein, diolein, triolein, monoglyceride stearate, diglyceride stearate, triglyceride stearate, monoglyceride palmitate, diglyceride palmitate, triglyceride palmitate, monoglyceride myristate, diglyceride myristate, triglyceride myristate, monoglyceride arachidate, diglyceride arachidate, triglyceride arachidate, monoglyceride behenate, diglyceride behenate, and triglyceride behenate;
[22] use of [20], wherein (ii) is one or two or more substances selected from the group consisting of linoleic acid, stearic acid, oleic acid, lauric acid, and palmitic acid;
[23] use of [20], wherein (iii) is one or two or more substances selected from the group consisting of polyoxyethylene polyoxypropylene glycol, polyoxyethylene fatty acid monoester, saturated polyglycosylated glyceride, D-α-tocopheryl polyethylene glycol 1000 succinate, and polyoxyethylene hydrogenated castor oil;
[24] use of [20], wherein (iv) is one or two or more substances selected from the group consisting of stearyl alcohol, myristyl alcohol, and cetyl alcohol;
[25] use of [20], wherein the lipophilic substance which inhibits IL-2 production has a LogP value of 2 or more and an IC₅₀ (a 50% inhibitory drug concentration in a human mixed lymphocyte reaction) of 1 µmol/L or less; and
[26] use of [20], wherein the lipophilic substance which inhibits IL-2 production is one or two or more substances selected from the group consisting of tacrolimus, cyclosporin, rapamycin, mycophenolic acid, and mycophenolate mofetil.
The present invention includes an embodiment in which tacrolimus and salts thereof are excluded as the lipophilic substance which inhibits IL-2 production.

### EFFECTS OF THE INVENTION

The present invention is **characterized in that**, when a lipophilic substance which inhibits IL-2 production is orally administered, a carrier capable of inhibiting blood exposure of the substance and of efficiently delivering the substance to lymph is selected and used. Therefore, the present invention is different from technologies for improving oral absorption to enhance bioavailability. Since it was confirmed that the pharmaceutical composition of the present invention could inhibit blood exposure of a lipophilic substance which inhibits IL-2 production and could develop its pharmacological effect, an immunosuppressive effect, decreased adverse effects such as abnormal renal function or the like are expected, and the present invention exhibits effects different from those of technologies that increase the absorption of a drug orally administered.

### BRIEF DESCRIPTION OF THE DRAWINGS

Figure 1 is a graph showing a cumulative degradation rate to fatty acids in samples containing 30 mg of medium chain triglycerides, sunflower oil, or cacao butter, and a sample containing 300 mg of sunflower oil.
Figure 2 is a graph showing a cumulative absorption rate of tacrolimus following oral administration of the compositions of the present invention prepared in Examples 1, 2, 3, and 5.
Figure 3 is a graph showing a correlation between degradation rates to fatty acids after the reaction for 1 hour, obtained in Experimental Example 2, and blood AUC₀₋₁₉ₕ of tacrolimus following administration of the formulations of the present invention prepared in Examples 1, 2, 3, and 5, obtained in Experimental Example 1.
Figure 4 is a graph showing a correlation between the content by weight of fatty acids having a melting point of 50°C or more contained in the molecular composition of a base and blood AUC₀₋₁₉ₕ of tacrolimus following administration of the formulations prepared in Examples 1, 4, and 6 and Comparative Example 1.
Figure 5 is a graph showing a lymphatic transfer (a value obtained by dividing a lymph transfer amount of tacrolimus by a blood AUC) following administration of each formulation.

### BEST MODE FOR CARRYING OUT THE INVENTION

The term "lipophilic substance which inhibits IL-2 production" as used herein means a compound which is lipophilic and inhibits IL-2 production, or a pharmaceutically acceptable salt thereof, more particularly, a compound having a LogP value [calculated using ACD/LogD Suite version 8.0 (Advanced Chemistry Development Inc.)] of 2 or more and an IC₅₀ (determined by test method-1 as described below) of 1 µmol/L or less, or a pharmaceutically acceptable salt thereof. The LogP value is a logarithm of a 1-octanol/water partition coefficient P to the base 10. The 1-octanol/water partition coefficient P is a value obtained by dividing a drug concentration in 1-octanol by a drug concentration in water, when a drug and a two-liquid-phase solvent of 1-octanol and water are mixed and equilibrated. A higher LogP value of a drug indicates that the drug has an affinity for 1-octanol rather than water, and is more lipophilic. The term "lipophilic" as used herein means a LogP value of 2 or more.

Test method-1 is as follows. As described in Transplant Proceedings, No 5, Suppl 6, 36-39, 1987, lymphocytes are separated by a density gradient method using Ficoll-Paque (Pharmacia Fine Chemicals) from fresh defibrinized blood taken from a healthy volunteer, and washed with a Hanks solution three times. The lymphocytes (5×10⁵ cells) are cultured in 0.2 mL of an RPMI 1640 complete medium at 37°C (CO₂ concentration = 5%, air = 95%) for 96 hours. This cultivation is carried out in the presence of 0.5 µCi tritiated thymidine (³H-TdR, 6.7Ci/nmol, New England Nuclear) for the last 4 hours. The culture sample is collected on a glass filter paper of a microharvester (Bellco Glass Inc.). The radioactivity of the material contained in the dried filter paper is measured with a liquid scintillation counter to calculate an amount of ³H-TdR incorporated into lymphocytes. An amount of ³H-TdR when the lymphocytes are cultured in the presence of a drug to be assayed is divided by an amount of ³H-TdR when the cultivation is performed without the drug, and multiplied by 100, and the resulting value is regarded as an uptake ratio (%). Concentrations of a drug to be added in the cultivation are varied, and a drug concentration at which the uptake ratio becomes 50% is regarded as an IC₅₀ (a 50% inhibitory drug concentration in a human mixed lymphocyte reaction). The term "lipophilic substance which inhibits IL-2 production" as used herein means a compound having an IC₅₀ (determined by test method-1) of 1 µmol/L or less, or a pharmaceutically acceptable salt thereof. The lipophilic substances which inhibit IL-2 production include, for example, tacrolimus, cyclosporin, rapamycin, mycophenolic acid, mycophenolate mofetil, and the like, and the substance is tacrolimus in another embodiment. These substances which inhibit IL-2 production may be used alone, or a mixture of two or more. For example, a combination of one or two or more substances selected from the group consisting of tacrolimus, mycophenolic acid and mycophenolate mofetil may be used. A combination of one or two or more substances selected from the group consisting of cyclosporin, rapamycin, mycophenolic acid and mycophenolate mofetil may be used in another embodiment. A combination of one or two or more substances selected from the group consisting of the substances which inhibits IL-2 production except tacrolimus may be used in still another embodiement.

Tacrolimus (LogP value = 3 or more) or a pharmaceutically acceptable salt thereof (hereinafter sometimes and simply referred to as "tacrolimus or a salt thereof") is disclosed in Japanese Unexamined Patent Publication (Kokai) No. 61-148181 (corresponding to European Patent Publication No. 0323042), and defined by the following chemical name or the following general formula.

[3S-[3R*[E(1S*,3S*,4S)],4S*,5R*,8S*,9E,12R*,14R*,15S*,16R*, 18S*,19S*,26aR*]]-5,6,8,11,12,13,14,15,16,17,18,19,24,25,26, 26a-hexadecahydro-5,19-dihydroxy-3-[2-(4-hydroxy-3-methoxycyclohexyl)-1-methylethenyl]-14,16-dimethoxy-4,10,12,18-tetramethyl-8-(2-propenyl)-15,19-epoxy-3H-pyrido[2,1.-c][1,4] oxaazacyclotricosine-1,7,20,21(4H,23H)-tetrone, monohydrate

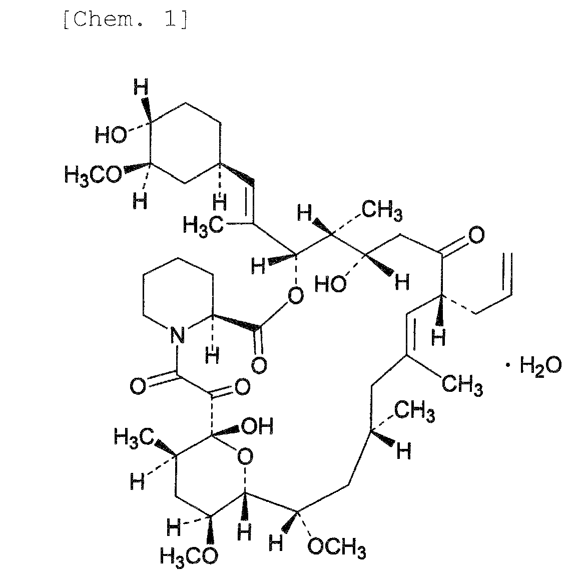

Tacrolimus or a salt thereof is useful for the treatment or prevention of the following diseases.
Autoimmune diseases of the eye (for example, keratoconjunctivitis, vernal conjunctivitis, uveitis associated with Behcet's disease, keratitis, herpetic keratitis, conical keratitis, corneal epithelial dystrophy, keratoleukoma, ocular premphigus, Mooren's ulcer, scleritis, Graves' ophthalmopathy, Vogt-Koyanagi-Harada syndrome, keratoconjunctivitis sicca (dry eye), phlyctenule, iridocyclitis, sarcoidosis, endocrine ophthalmopathy, or the like);
skin diseases [for example, dermatomyositis, leukoderma vulgaris, ichthyosis vulgaris, photosensitivity, cutaneous T-cell lymphoma, inflammatory or hyperproliferative skin diseases, cutaneous manifestations of immunologically-mediated diseases (such as atopic dermatitis, contact dermatitis, eczematoid dermatitis, seborrheic dermatitis, lichen planus, pemphigus, bullous pemphigoid, epidermolysis bullosa, urticaria, angioedema, vasculitides, erythema, dermal eosinophilia, lupus erythematosus, acne, alopecia areata, and the like), or psoriasis (such as psoriasis arthropathica, psoriasis circinata, psoriasis diffusa, psoriasis discoidea, generalized pustular psoriasis of Zumbusch, psoriasis geographica, psoriasis guttata, psoriasis gyrata, psoriasis inveterata, psoriasis nummularis, psoriasis orbicularis, psoriasis ostreacea, psoriasis punctata, pustular psoriasis, psoriasis spondylitica, psoriasis universalis, or the like); hypertrophic cicatrix or keloid due to trauma, burn, surgery, or the like;
rejection reactions by transplantation of organs or tissues such as the heart, kidney, liver, bone marrow, skin, cornea, lung, pancreas, small intestine, limb, muscle, nerve, intervertebral disc, trachea, myoblast, cartilage, or the like;
graft-versus-host reactions following bone marrow transplantation;
rheumatoid arthritis, systemic lupus erythematosus, Hashimoto's thyroiditis, multiple sclerosis, myasthenia gravis, type I diabetes, or the like;
infections caused by pathogenic microorganisms (such as Aspergillus fumigatus, Fusarium oxysporum, Trichophyton asteroides, or the like);
reversible obstructive airways diseases [asthma (such as bronchial asthma, allergic asthma, intrinsic asthma, extrinsic asthma, or dust asthma), particularly chronic or inveterate asthma (such as late asthma or airway hyper-responsive bronchitis), or the like];
mucosal or vascular inflammations (such as gastric ulcer, ischemic or thrombotic vascular injury, ischemic bowel diseases, enteritis, necrotizing enterocolitis, intestinal damages associated with thermal burns, leukotriene B4-mediated diseases);
intestinal inflammations/allergies (such as coeliac diseases, proctitis, eosinophilic gastroenteritis, mastocytosis, Crohn's disease, or ulcerative colitis); food-related allergic diseases with symptomatic manifestation remote from the gastrointestinal tract (such as migrain, rhinitis, or eczema);
renal diseases (such as intestitial nephritis, Goodpasture's syndrome, hemolytic uremic syndrome, or diabetic nephropathy);
nervous diseases (such as multiple myositis, Guillain-Barre syndrome, Meniere's disease, multiple neuritis, solitary neuritis, cerebral infarction, Alzheimer's disease, Parkinson's disease, amyotrophic lateral sclerosis (ALS), or radiculopathy);
cerebral ischemic disease [such as head injury, hemorrhage in brain (such as subarachnoid hemorrhage, intracerebral hemorrhage), cerebral thrombosis, cerebral embolism, cardiac arrest, stroke, transient ischemic attack (TIA), hypertensive encephalopathy, cerebral infarction]; endocrine diseases (such as hyperthyroidism, or Basedow's disease); hematic diseases (such as pure red cell aplasia, aplastic anemia, hypoplastic anemia, idiopathic thrombocytopenic purpura, autoimmune hemolytic anemia, agranulocytosis, pernicious anemia, megaloblastic anemia, or anerythroplasia);
bone diseases (such as osteoporosis);
respiratory diseases (such as sarcoidosis, pulmonary fibrosis, or idiopathic interstitial pneumonia);
skin diseases (such as dermatomyositis, leukoderma vulgaris, ichthyosis vulgaris, photosensitivity, or cutaneous T-cell lymphoma);
circulatory diseases (such as arteriosclerosis, atherosclerosis, aortitis syndrome, polyarteritis nodosa, or myocardosis);
collagen diseases (such as scleroderma, Wegener's granuloma, or Sjogren's syndrome);
adiposis;
eosinophilicfasciitis;
periodontal diseases (such as damage to gingiva, periodontium, alveolar bone, or substantia ossea dentis);
nephrotic syndrome (such as glomerulonephritis);
male pattern alopecia, alopecia senile;
muscular dystrophy;
pyoderma or Sezary syndrome;
chromosome abnormality-associated diseases (such as Down's syndrome); or
Addison's disease.

Cyclosporin which may be used in the present invention is the following cyclosporin or cyclosporins. Cyclosporin (LogP value = 2.92) or a pharmaceutically acceptable salt thereof (hereinafter sometimes and simply referred to as "cyclosporin or a salt thereof") is defined by the following chemical name or the following general formula.

[R-[[R*,R*-(E)]]-cyclic(L-alanyl-D-alanyl- N-methyl-L-leucyl-N-methyl-L-leucyl- N-methyl-L-valyl-3-hydroxy-N,4-dimethyl-L-2-amino-6-octenoyl-L-a-amino-butyryl-N-methylglycyl-N-methyl-L-leucyl-L-valyl-N-methyl-L-leucyl) 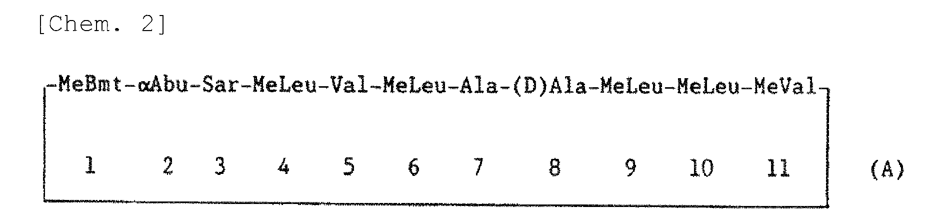 [wherein -MeBmt- is an N-methyl-(4R)-4-but-2E-en-1-yl-4-methyl-(L)threonyl residue of the formula (B) 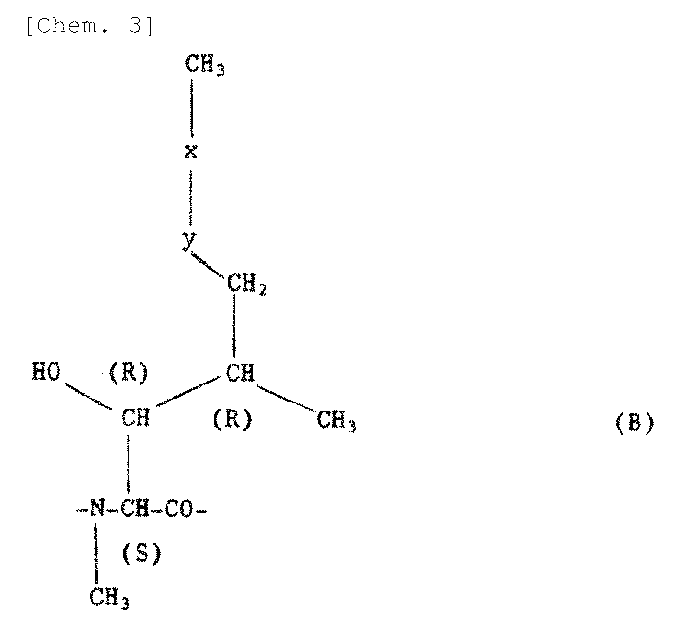 (wherein -x-y- is -CH=CH- (trans)).]

Cyclosporins include naturally occurring cyclosporins A to Z [see, Traber et al., 1, Helv.Chim.Acta. 60, 1247-1255(1977), Traber et al., 2, Helv.Chim.Acta. 65, No. 162, 1655-1667(1982), Kobel et al., Europ.J.Applied Microbiology and Biotechnology 14, 273-240(1982), and von Wartburg et al., Progress in Allergy, 38, 28-45(1986)] and various nonnatural cyclosporin derivatives, and artificial or synthetic cyclosporins, such as so-called dihydro-cyclosporins [in this case, the -x-y- portion of the - MeBmt- residue (above formula B) is saturated to obtain -x-y-=-CH₂-CH₂-], derivatized cyclosporins (for example, another substituent group is incorporated into the α-carbon atom of the sarkosyl residue at the 3-position of a cyclosporin molecule), cyclosporins in which the -MeBmt-residue exists in an isomeric form (for example, the configuration between the 6' and 7' positions in the - MeBmt- residue is not trans but cis), and cyclosporins in which one or more modified amino acids are incorporated into specific positions of a peptide sequence by, for example, a total synthetic process of manufacturing cyclosporins developed by Wenger [see, for example, Traber 1, Traber 2, Kobel, as described above, U.S. Patent No. 4108985, U.S. Patent No. 4210581, U.S. Patent No. 4220641, European Patent Publication No. 0034567, European Patent Publication No. 0056782, European Patent Publication No. 0296122, International Publication No. WO86/02080, Wenger 1, Transp.Proc. 15, suppl.1:2230(1983), Wenger 2, Angew.Chem.Int.Ed., 24, 77(1985), and Wenger 3, Progress in the Chemistry of Organic Natural Products, 50, 123(1986)].

As cyclosporins, there may be mentioned, for example, [Thr]2-, [Val]2-, [Nva]2-, and [Nva]2-[Nva]5-Ciclosporin (known as cyclosporins C, D, G, and M, respectively), [3'-O-acetyl-MeBmt]1-Ciclosporin (also known as cyclosporin A acetate), dihydro-[MeBmt]1-[Val]2-Ciclosporin (known as dihydro-cyclrosporin D), 3'-desoxy-3'-oxo-[MeBmt]1-[Val]2-and -[Nva]2-Ciclosporin, [(D)fluoromethyl-Sar]3-Ciclosporin, [(D)Ser]8-Ciclosporin, [MeIle]11-Ciclosporin, [(D)MeVal]11-Ciclosporin (known as cyclosporin H), [MeAla]6-Ciclosporin, [(D)Pro]3-Ciclosporin, and the like. [In accordance with now conventional nomenclature for cyclosporins, these are defined by reference to the structure of Ciclosporin (i.e., Cyclosporin A). This is done by first indicating the amino acid residues present which differ from those present in Ciclosporin (for example, "[(D)Pro]3" to indicate that the cyclosporin in question has a -(D)Pro- rather than -Sar- residue at the 3-position) and then applying the term "Ciclosporin" to characterise remaining residues which are identical to those present in Ciclosporin. Individual residues are numbered starting with the residue -MeBmt-, -dihydro-MeBmt-or its equivalent in position 1.]

A great many cyclosporins exhibit comparable pharmaceutical utility to cyclosporin or more specific utility, for example, activity particularly in reversing tumor resistance to cytostatic therapy, and many proposals for their application as therapeutic agents are described in the literature.

Cyclosporin or a salt thereof is useful for the treatment or prevention of the following diseases.
Rejection reactions following organ transplants, such as heart, lung, combined heart-lung, liver, kidney, pancreatic, bone-marrow, skin, and corneal transplants and, in particular, allogenic organ transplants;
autoimmune disease and inflammatory conditions, particularly inflammatory conditions with an aetiology including an autoimmune component such as arthritis (for example, rheumatoid arthritis, arthritis chronica progrediente, arthritis deformans) and rheumatic diseases; autoimmune haematological disorders (including, for example, hemolytic anaemia, aplasticanaemia, pure red cell anaemia, and idiopathic thrombocytopenia), systemic lupus erythematosus, polychondritis, sclerodoma, Wegener granulamatosis, dermatomyositis, chronic active hepatitis, myasthenia gravis, psoriasis, Steven-Johnson syndrome, idiopathic sprue, autoimmune inflammatory bowel disease (including, for example, ulcerative colitis and Crohn's disease), endocrineophthalmopathy, Graves disease, sarcoidosis, multiple sclerosis, primary biliary cirrhosis, juvenile diabetes (diabetes mellitus type I), uveitis (anterior and posterior), keratoconjunctivitis sicca and vernal keratoconjunctivitis, interstitial lung fibrosis, psoriatic arthritis and glomerulonephritis (with and without nephrotic syndrome, for example, including idiopathic nephrotic syndrome or minimal change nephropathy);
diseases due to parasites, particularly protozoa, such as malaria, coccidiomycosis, and schistosomiasis; and use in cancer therapy as an agent for revising or abrogating resistance to other anti-neoplastic or cytostatic therapy.

Rapamycin or a pharmaceutically acceptable salt thereof (hereinafter sometimes and simply referred to as rapamycin or a salt thereof) which may be used in the present invention is defined by the following chemical name.

(3S,6R,7E,9R,10R,12R,14S,15E,17E,19E,21S,23S,26R,27R,34aS)-9,10,12,13,14,21,22,23,24,25,26,27,32,33,34,34a-hexadecahydro-9,27-dihydroxy-3-[(1R)-2-[(1S,3R,4R)-4-hydroxy-3-methoxycyclohexyl]-1-methylethyl]-10,21-dimethoxy-6,8,12,14,20,26-hexamethyl-23,27-epoxy-3H-pyrido[2,1-c][1,4]-oxaazacyclohentriacontine-1,5,11,28,29(4H,6H,31H)-pentone

Rapamycin or a salt thereof is useful for the treatment or prevention of the following diseases.
Mycoses, particularly mycosis due to Candida albicans; allergic encephalomyelitis, multiple sclerosis, arthritis, chronic rheumatism;
rejection reactions following transplantation of organs or tissues such as the heart, kidney, liver, bone marrow, skin, cornea, lung, pancreas, small intestine, limb, muscle, nerve, intervertebral disc, trachea, myoblast, cartilage, or the like;
graft-versus-host reactions following bone marrow transplantation;
systemic lupus erythematosus, pneumonia, insulin-dependent diabetes mellitus, proliferation of smooth muscle cells and intimal thickening following vascular injury, adult T-cell leukaemia/lymphoma, and ocular inflammation.

Mycophenolic acid or a pharmaceutically acceptable salt thereof (hereinafter sometimes and simply referred to as mycophenolic acid or a salt thereof) which may be used in the present invention was initially described as a weakly-active antibiotic found in a fermentation broth of penicillium brevicompactum, and is defined by the following chemical name or the following general formula.

(E)-6-(4-hydroxy-6-methoxy-7-methyl-3-oxo-1,3-dihydroisobenzofuran-5-yl)-4-methylhex-4-enoic acid

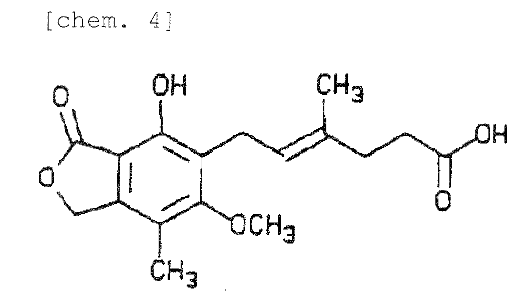

Mycophenolate mofetil (LogP value = 2.38) or a pharmaceutically acceptable salt thereof (hereinafter sometimes and simply referred to as "mycophenolate mofetil or a salt thereof") which may be used in the present invention is described in EP281713B1 and is defined by the following chemical name or the following general formula.

2-morpholinoethyl (E)-6-(1,3-dihydro-4-hydroxy-6-methoxy-7-methyl-3-oxo-5-isobenzofuranyl)-4-methyl-4-hexenoate

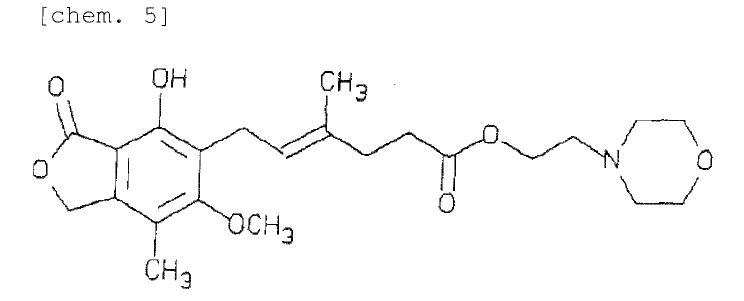

Mycophenolic acid or a salt thereof and mycophenolate mofetil or a salt thereof are useful for the treatment or prevention of the following diseases.

As immunosuppressive agents, the compounds are useful in treating auto-immune related disorders, for example: Type I Diabetes Mellitus; Inflammatory Bowel Disease (e.g., Crohn's Disease and Ulcerative Colitis); Systemic Lupus Erythematosus; Cronic Active Hepatitis; Multiple Sclerosis; Grave's Disease; Hashimoto's Thyroiditis; Behcet's Syndrome; Myasthenia Gravis; Sjogren's Syndrome; Pernicious Anemia; Idiopathic Adrenal Insufficiency; and Polyglandular Autoimmune Syndrome Type I and II.

The compounds are also useful as therapeutic immunosuppressive agents in the treatment of Asthma, Immunohemolytic Anemia, Glomerulonephritis, and Hepatitis. Preventative uses of the compounds as immunosuppressive agents include the treatment of allograft rejection, for example, in cardiac, lung, pancreatic, renal, liver, skin, and corneal allografts, and prevention of Graft versus Host Disease.

The compounds are useful for inhibiting proliferative responses to vascular injury, for example, stenosis following an insult to a blood vessel wall in post-angioplasty restenosis, and post-cardiac by-pass surgery restenosis.

The compounds are useful as anti-inflammatory agents, for example, in treating Rheumatoid Arthritis, Juvenile Rheumatoid Arthritis and Uveitis.

As anti-tumor agents, the compounds are useful in treating solid tumors and malignancies of lymphoreticular origin. For example, the compounds utility for treatment of solid tumors includes: cancers of the head and neck, including squamous cell carcinoma; lung cancer, including small cell and non-small cell lung carcinoma; mediastinal tumors; esophageal cancer including squamous cell carcinoma and adenocarcinoma; pancreatic cancer; cancer of the hepatobiliary system, including hepatocellular carcinoma, cholangiocarcinoma, gall bladder carcinoma and biliary tract carcinoma; small intestinal carcinoma including adenocarcinoma, sarcoma, lymphoma and carcinoids; colorectal cancer, including colon carcinoma and rectal carcinoma; metastatic carcinoma; cancers of the genitourinary system, including ovarian cancer, uterine sarcoma, and renal cell, ureteral, bladder, prostate, urethral, penile, testicular, vulvar, vaginal, cervical, endometrial, and fallopian tube carcinoma; breast cancer; endocrine system cancer; soft tissue sarcomas; malignant mesotheliomas; skin cancer, including squamous cell carcinoma, basal cell carcinoma and melanoma; cancer of the central nervous system; malignant bone tumors; and plasma cell neoplasms.

As anti-tumor agents for the treatment of malignancies of lymphoreticular origin, the compounds are useful in treating, for example: Lymphomas and Leukemias, including B, T and promonocyte cell line malignancies, Mycoses Fungoides, Non-Hodgkins Lymphoma, Malignancies of Burkitt Lymphoma Cells and other EBV-transformed B-lymphocytes, Lymphomas resulting from Epstein-Barr viral infections in allograft recipients, Chronic Lymphocytic Leukemia, Acute Lymphocytic Leukemia and Hairy Cell Leukemia.

As anti-viral agents, the compounds are useful in treating, for example: retroviruses, including Human T-leukemia Viruses, Type I and II (HTLV-1, HTLV-2), Human Immuno Deficiency Viruses, Type I and II (HIV-I, HIV-II), and Human Nasopharyngeal Carcinoma Virus (NPCV), and in treating Herpes Viruses, including EBV infected B-lymphocytes, CMV infection, Herpes Virus Type 6, Herpes Simplex, Type 1 and 2 (HSV-1,HSV-2) and Herpes Zoster.

As anti-psoriatic agents, the compounds are useful in treating, for example, psoriasis and psoriatic arthritis.

The content of a lipophilic substance which inhibits IL-2 production is not particularly limited, so long as it may be appropriately adjusted in accordance with the age of a patient, the type or symptoms of a disease, an amount effective for prevention, or other factors. A daily dose is generally approximately 0.05 to 3000 mg, and 0.05 to 2000 mg in another embodiment. A daily dose of the substances which inhibit IL-2 production except mycophenolic acid and mycophenolate mofetil is 0.05 to 500 mg in another embodiement, and 0.1 to 100 mg in still another embodiement. As a single dose, 0.1 mg, 0.5 mg, 1 mg, 5 mg, 10 mg, 50 mg, 100 mg, 500 mg, or 1000 mg may be generally administered on average (as an amount of a lipophilic substance which inhibits IL-2 production when provided as a hydrate). Administration may be generally carried out twice a day.

A lipophilic substance which inhibits IL-2 production generally accounts for 0.005 to 90 w/w%, and 0.05 to 50 w/w% in another embodiment, with respect to the total amount of the pharmaceutical composition of the present invention.

A carrier capable of inhibiting the transfer of a lipophilic substance which inhibits IL-2 production to blood and of delivering the substance to lymph (hereinafter referred to as "a carrier for delivering a lipophilic substance which inhibits IL-2 production to lymph"), which may be used in the present invention, is not particularly limited, so long as it is pharmaceutically acceptable, and can inhibit the transfer of a lipophilic substance which inhibits IL-2 production to blood and efficiently deliver the substance to lymph, after oral administration. As the carrier, for example, a substance having properties described in item (I) described below, substances described in items (i) to (iv) described below, or the like may be used. These substances may be used alone or as a mixture of two or more substances.

As the carrier, used in the present invention, for delivering a lipophilic substance which inhibits IL-2 production to lymph, an embodiment in which a lower alcohol such as ethanol or the like is not substantially contained may be selected. The term "lower alcohol" as used herein means an alcohol having 1 to 5 carbon atoms. The present invention does not exclude an embodiment containing a lower alcohol in an amount which does not affect the inhibition of a transfer of a lipophilic substance which inhibits IL-2 production to blood nor the lymphatic delivery thereof.

The term "lymphatic delivery" as used herein means the property that blood exposure of a lipophilic substance which inhibits IL-2 production is inhibited; and when contents of the world's first commercially available capsule formulation containing a lipophilic substance which inhibits IL-2 production [for example, tacrolimus hydrate capsule containing tacrolimus (such as Prograf capsule, Astellas Pharma), cyclosporin capsule containing cyclosporin A (such as Sandimmun capsule, Novartis Pharma), or mycophenolate mofetil capsule containing mycophenolate mofetil (such as Celcept capsule, Roche)] are used as a control composition, a lymph AUC_{lymph} determined by test method-2 described below is 50% to 200% compared to that of the control.

The term "lymphatic delivery" as used herein means the property that blood exposure of a lipophilic substance which inhibits IL-2 production is inhibited; and an IL-2 production rate determined by test method-3 described below is 0% to 40%, 0% to 30% in another embodiment, or 0% to 20% in still another embodiment.

The term "lymphatic delivery" as used herein means the property that blood exposure of a lipophilic substance which inhibits IL-2 production is inhibited; and when contents of the world's first commercially available capsule formulation containing a lipophilic substance which inhibits IL-2 production [for example, tacrolimus hydrate capsule containing tacrolimus (such as Prograf capsule, Astellas Pharma), cyclosporin capsule containing cyclosporin A (such as Sandimmun capsule, Novartis Pharma), or mycophenolate mofetil capsule containing mycophenolate mofetil (such as Celcept capsule, Roche)] are used as a control composition, a concentration of a lipophilic substance which inhibits IL-2 production, included in 10⁷ lymph cells, determined by test method-7 described below is 50% to 200% to that of the control.

The term "inhibition of blood exposure" as used herein means the property that a blood AUC_{blood} determined by test method-4 described below is 0 ng·h/mL to 90 ng·h/mL, 0 ng·h/mL to 70 ng·h/mL in another embodiment, or 0 ng·h/mL to 50 ng·h/mL in still another embodiment; or the property that a blood AUC_{blood} determined by test method-5 described below is 0 ng·h/mL to 140 ng·h/mL, 0 ng·h/mL to 110 ng·h/mL in another embodiment, or 0 ng·h/mL to 80 ng·h/mL in still another embodiment.

The term "inhibition of blood exposure" as used herein means the property that when contents of the world's first commercially available capsule formulation containing a lipophilic substance which inhibits IL-2 production [for example, tacrolimus hydrate capsule containing tacrolimus (such as Prograf capsule, Astellas Pharma), or cyclosporin capsule containing cyclosporin A (such as Sandimmun capsule, Novartis Pharma)] are used as a control composition, a blood AUC_{blood} determined by test method-4 or test method-5 described below is 0% to 80%, 0% to 60% in another embodiment, or 0% to 40% in still another embodiment, with respect to the control.

The term "inhibition of blood exposure" as used herein means the property that when contents of the world's first commercially available capsule formulation containing a lipophilic substance which inhibits IL-2 production [for example, mycophenolate mofetil capsule containing mycophenolate mofetil (such as Celcept capsule, Roche)] are used as a control composition, a plasma AUCₚₗₐₛₘₐ determined by test method-4 described below is 0% to 80%, 0% to 60% in another embodiment, or 0% to 40% in still another embodiment, with respect to the control.

The term "test method-2" as used herein means the following test. The thoracic lymph duct is cannulated in a rat (male Lewis rat, 300-350 g) which has been fasted for 12 hours or more, and it is confirmed that lymph can be stably collected. A test liquid is forcefully orally administered using a catheter to the rat. The rat is fasted until lymph collections following the administration are completed, but is allowed to freely take water. Concentrations of a lipophilic substance which inhibits IL-2 production contained in lymph samples collected during predetermined periods of time (0-1, 1-2, 2-3, 3-4, 4-6, and 6-24 hours after the administration) are measured by LC-MS/MS, HPLC, or the like. The area under the lymph concentration versus time curve (lymph AUC_{lymph}) is calculated by the trapezoidal rule, as the substance concentration at the midpoint of the period of lymph collection.

The term "test method-3" as used herein means the following test. A test liquid is forcefully orally administered using a catheter to a rat (male Lewis rat, 6 weeks) which has been fasted for 12 hours or more. The rat is fasted until a blood collection following the administration, but is allowed to freely take water. Concanavalin A (Funakoshi Co., Ltd) is added to physiological saline at a concentration of 1 mg/mL, and the whole is stirred for 5 minutes and passed through a filter of 0.45 µm (NALGENE, SFCA Filter Unit) to obtain a filtrate (hereinafter referred to as concanavalin A solution). After 16 hours have passed from the administration of the test liquid to the rat, the concanavalin A solution (within 2 hours from the preparation) is rapidly intravenously administered to the rat, at a dose of 15 mg/kg of concanavalin A. Blood samples are collected after 3 hours from the administration of the concanavalin A solution, and centrifuged to obtain plasma samples. An ELISA kit (BioSource International, Inc., Rat IL-2 ELISA kit) is used to measure plasma concentrations of IL-2. Similarly, the concanavalin A solution is rapidly intravenously administered to a rat to which no test liquid has been administered (hereinafter referred to as the untreated group); blood samples are collected after 3 hours from the administration, and centrifuged to obtain plasma samples; and plasma concentrations of IL-2 are measured by the ELISA kit (BioSource International, Inc., Rat IL-2 ELISA kit) .
The percentage (%) of a plasma concentration of IL-2 in a rat to which the test liquid is administered with respect to that in the untreated group is regarded as a rate of IL-2 production.

The term "test method-4" as used herein means the following test. A test liquid is forcefully orally administered to a rat (male Lewis rat, 6 weeks) which has been fasted for 12 hours or more. The rat is fasted until a blood collection following the administration, but is allowed to freely take water. Blood samples are collected after 1, 4, 8, 12, and 19 hours from the administration. Whole blood concentrations of a lipophilic substance which inhibits IL-2 production are measured by LC-MS/MS, HPLC, or the like, and the area under the blood concentration versus time curve (blood AUC_{blood}) is calculated by the trapezoidal rule. Alternatively, in case of a lipophilic substance which inhibits IL-2 production and shows enterohepatic circulation (for example, mycophenolic acid, mycophenolate mofetil, or the like), blood samples are collected after 0.5, 1 and 2 hours from the administration, and centrifuged to obtain plasma samples. Plasma concentrations of mycophenolic acid were measured by HPLC, and an area under the plasma concentration versus time curve (plasma AUCₚₗₐₛₘₐ) is calculated.

The term "test method-5" as used herein means the following test. A capsule for administration is forcefully orally administered, together with 10 mL of water, to a cynomolgus monkey (male, 3-5 years, 2.8-3.8 kg) which has been fasted for 16 hours or more. The monkey is fasted until a blood collection after 24 hours from the administration is completed, but is allowed to freely take water. Blood samples are collected before the administration, and after 0.5, 1, 2, 4, 6, 8, 12, 24, and 48 hours from the administration. Whole blood concentrations of a lipophilic substance which inhibits IL-2 production are measured by LC-MS/MS, HPLC, or the like, and the area under the blood concentration versus time curve (blood AUC_{blood}) is calculated by the trapezoidal rule.

The term "test method-6" as used herein means the following test. Sodium taurocholate is dissolved in a phosphate buffer (pH6.8) (Japanese Pharmacopeia, Fourteenth Edition, a disintegration test, the second fluid) at a concentration of 50 mmol/L. Lipase (SIGMA, Lipase from porcine pancreas, 329 U/mg) is added thereto at a concentration of 375 U/mL, and stirred for 2 hours to prepare simulated intestinal fluid. As samples before a reaction at approximately 28°C while shaking (YAMATO SCIENTIFIC CO., LTD., maximum speed), 30 mg or 300 mg of a base is added to 6.0 mL of the simulated intestinal fluid. As samples after the reaction, these samples are shaken at approximately 28°C for 0.5, 1, or 3 hours. A kit for measuring nonesterified fatty acids (Wako Pure Chemical Industries, Ltd., NEFA C Test Wako) is used to measure concentrations of nonesterified fatty acids contained in the samples before the reaction and the samples after the reaction. Further, fatty acid compositions described in the Handbook of Oil Chemistry (Revised 3rd ed., Japan Oil Chemists' Society, Maruzen Co., Ltd.) are used to calculate theoretical concentrations of fatty acids generated when the base is completely degraded. The percentage (%) of the difference between the concentration of nonesterified fatty acids contained in a sample after the reaction and a concentration of nonesterified fatty acids contained in a sample before the reaction with respect to a theoretical concentration of fatty acids is regarded as a degradation rate to fatty acids.

The term "test method-7" as used herein means the following test. A test liquid is forcefully orally administered to a rat (male Lewis rat, 6 weeks) which has been fasted for 12 hours or more. The rat is fasted until a blood collection following the administration, but is allowed to freely take water. Whole blood samples are collected after 16 hours from the administration. Lymph cells are isolated utilizing a Ficcoll-conray solution (Lymphosepar II, Immuno-Biological Laboratories Co., Ltd.) by the density gradient method of sucrose. A concentration of a lipophilic substance which inhibits IL-2 production contained in 10⁷ lymph cells is measured by LC-MS/MS, HPLC, or the like.

The term "test liquid" means a solution prepared by dissolving or dispersing a lipophilic substance which inhibits IL-2 production in a carrier for delivering the lipophilic substance to lymph, or a suspension prepared by dissolving the lipophilic substance in the carrier, and further suspending the resulting composition at a concentration of 0.5% by weight, in a 0.5% by weight methylcellulose (Shin-Etsu Chemical Co., Ltd., SM-400) aqueous solution.

The carrier for delivering a lipophilic substance which inhibits IL-2 production to lymph, which may be used in the present invention, may be exemplified the following items (I) and (i) to (iv).

(I) A base which inhibits blood exposure of a lipophilic substance which inhibits IL-2 production, and has an IL-2 production rate of 0% to 40%, 0% to 30% in another embodiment, or 0% to 20% in still another embodiment.

(i) A mono-, di-, or tri-glyceride, or a mixture thereof. For example, rapeseed oil, castor oil, sunflower oil, cacao butter, medium chain triglycerides, monoolein, diolein, triolein, monoglyceride stearate, diglyceride stearate, triglyceride stearate, monoglyceride palmitate, diglyceride palmitate, triglyceride palmitate, monoglyceride myristate, diglyceride myristate, triglyceride myristate, monoglyceride arachidate, diglyceride arachidate, triglyceride arachidate, monoglyceride behenate, diglyceride behenate, and triglyceride behenate, may be exemplified. In another embodiment, sunflower oil, cacao butter, monoolein, medium chain triglycerides, monoglyceride stearate, diglyceride stearate, and triglyceride stearate, may be exemplified. The Handbook of Oil Chemistry (P.104-112, Revised 3rd ed., Japan Oil Chemists' Society, Maruzen Co., Ltd.) discloses typical fatty acid compositions of various fats and oils [see Table 1: "Fatty acid compositions (%) in various fats and oils"]. As commercially available medium chain triglycerides (product names), there may be mentioned, for example, ODO, Miglyol, Coconad, Panacet, Captex, Mynitol, Capmul, Neobee, Mazol, and the like. As commercially available monoglyceride stearate (product names), there may be mentioned, for example, MGS-F20V, MGS-F50V, MGS-50MV, MGS-F75V, MGS-75MV, IMWITOR 491, IMWITOR 900K, and the like. These compounds may be used alone, or a mixture of two or more compounds. It may be a base having a degradation rate to fatty acids after the reaction for 1 hour (determined by the above test method-6) of 0% to 60%, 0% to 50% in another embodiment, or 0% to 40% in still another embodiment; and a rate of IL-2 production (determined by the above test method-3) of 0% to 40%, 0% to 30% in another embodiment, or 0% to 20% in still another embodiment. In another embodiment, it may be a base in which the content by weight of fatty acids having a melting point of 50°C or more to total fatty acids contained in the molecular structure of the base is 10% to 100%, 20% to 100% in another embodiment, or 30% to 100% in still another embodiment; and a rate of IL-2 production (determined by the above test method-3) of 0% to 40%, 0% to 30% in another embodiment, or 0% to 20% in still another embodiment.

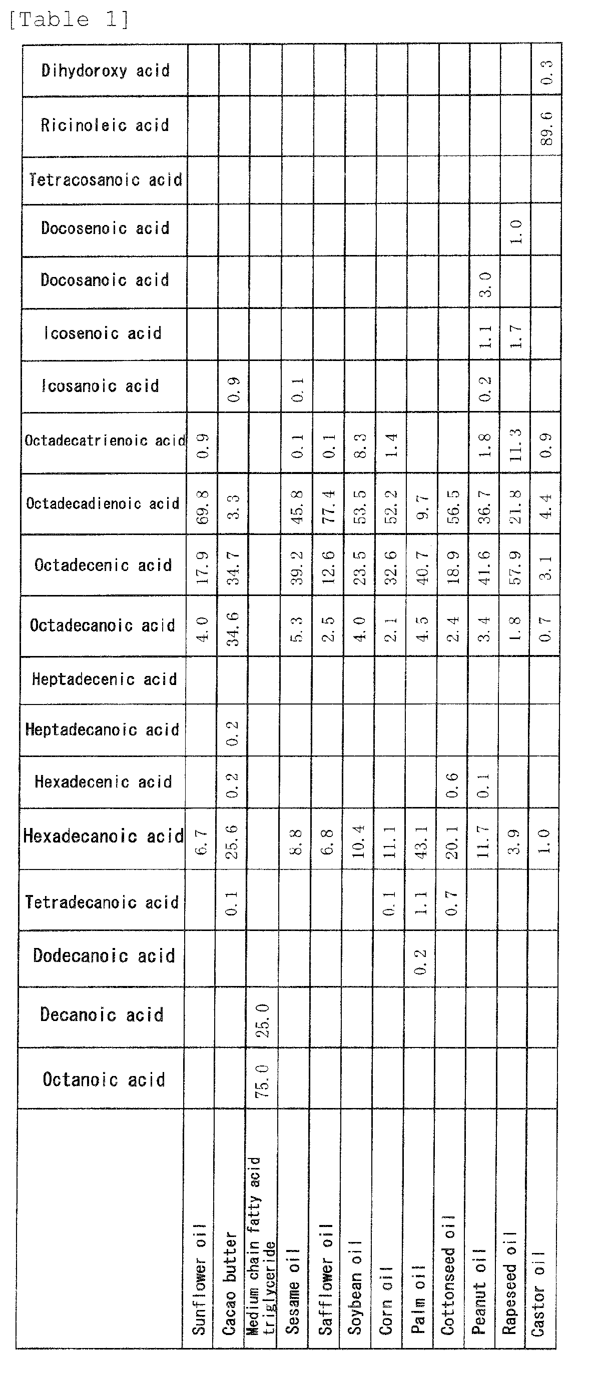

(ii) A fatty acid having 12 to 18 carbon atoms. For example, linoleic acid, stearic acid, oleic acid, lauric acid, palmitic acid, and the like may be exemplified. In another embodiment, oleic acid, lauric acid, and palmitic acid may be exemplified. These compounds may be used alone, or as a mixture of two or more compounds. Saturated or unsaturated fatty acids may be used.

(iii) A surfactant having an HLB value of 10 to 18 and containing polyethylene glycol having a weight average molecular weight of 200 to 5000 in its molecular structure. For example, polyoxyethylene polyoxypropylene glycol (weight average molecular weight: 1000 to 5000, content of a polyoxyethylene group: 10% to 50%, product name: Pluronic L44, Pluronic P85), polyoxyethylene fatty acid monoester (product name: Lipopeg 4-L), saturated polyglycosylated glyceride (product name: Gelucire 50/13), D-α-tocopheryl polyethylene glycol 1000 succinate (product name: TPGS), and polyoxyethylene hydrogenated castor oil (product name: HCO) are exemplified. In another embodiment, polyoxyethylene polyoxypropylene glycol (weight average molecular weight: 1000 to 5000, content of a polyoxyethylene group: 10% to 50%, product name: Pluronic L44, Pluronic P85), and saturated polyglycosylated glyceride (product name: Gelucire 50/13) may be exemplified. These surfactants may be used alone, or as a mixture of two or more surfactants.

(iv) A higher alcohol having 12 to 18 carbon atoms. For example, stearyl alcohol, myristyl alcohol, cetyl alcohol, and the like may be exemplified. These compounds may be used alone, or as a mixture of two or more compounds.

The contents of a lipophilic substance which inhibits IL-2 production and the carrier for delivering a lipophilic substance which inhibits IL-2 production to lymph, which may be used in the present invention, are not particularly limited, so long as the lipophilic substance which inhibits IL-2 production can be delivered to lymph. For example, the content of the carrier is 0.1 to 20,000 parts by weight, 1 to 10,000 parts by weight in another embodiment, or 10 to 2,000 parts by weight in still another embodiment, with respect to 1 part by weight of the lipophilic substance. When the content of the carrier is low, sometimes an absorption rate of a certain lipophilic substance which inhibits IL-2 production cannot be adequately controlled, and thereby blood exposure thereof tends not to be inhibited. To avoid this, the carrier is preferably contained in an amount higher than a certain content. Generally, these contents may be appropriately adjusted in accordance with a base. More particularly, 1 or more part by weight of monoglycerides having a melting point of 85°C or less as the carrier may be contained, with respect to 1 part by weight of a lipophilic substance which inhibits IL-2 production. In another embodiment, 10 or more parts by weight of triglycerides having a melting point of 25°C or less as the carrier may be contained, with respect to 1 part by weight of a lipophilic substance which inhibits IL-2 production. In still another embodiment, 100 or more parts by weight of fatty acids having a melting point of 25°C or less as the carrier may be contained, with respect to 1 part by weight of a lipophilic substance which inhibits IL-2 production.

The pharmaceutical composition of the present invention may be formulated using one or more appropriate pharmaceutical fillers, which are not particularly limited, so long as they are pharmaceutically and pharmacologically acceptable. Such fillers include, for example, emulsifiers, binders, disintegrating agents, acidulants, foaming agents, artificial sweeteners, flavors, lubricants, coloring agents, stabilizers, buffers, antioxidants, and the like.
As the emulsifiers, there may be mentioned, for example, reaction products of untreated or hydrogenated castor oil and ethylene oxide (product name: Cremophor, NIKKOL, MAPEG, INCROCAS, TAGAT), polyoxyethylene sorbitan fatty acid ester (product name: TWEEN), polyoxyethylene fatty acid ester (product name: MYRJ), polyoxyethylene-polyoxypropylene copolymer and block copolymer (product name: PLURONIC, EMKALYX, POLOXAMER), phospholipids (soybean lecithin), propylene glycol mono- and di- fatty acid ester (MIGLYOL), and the like. As the binders, there may be mentioned, for example, hydroxypropylmethyl cellulose, hydroxypropyl cellulose, polyvinyl alcohol, methyl cellulose, gum arabic, and the like. As the disintegrating agents, there may be mentioned, for example, corn starch, starches, carmellose calcium, carmellose sodium, low substituted hydroxypropylcellulose, and the like. As the acidulants, there may be mentioned, for example, citric acid, tartaric acid, malic acid, and the like. As the foaming agents, there may be mentioned, for example, sodium bicarbonate and the like. As the artificial sweeteners, there may be mentioned, for example, saccharin sodium, dipotassium glycyrrhizinate, aspartame, stevia, somatin, and the like. As the flavors, there may be mentioned, for example, lemon, lemon lime, orange, menthol, and the like. As the lubricants, there may be mentioned, for example, magnesium stearate, calcium stearate, sucrose fatty acid ester, polyethylene glycol, talc, stearic acid, and the like. As the coloring agents, there may be mentioned, for example, yellow ferric oxide, red ferric oxide, food yellow No. 4, food yellow No. 5, food red No. 3, food red No. 102, food blue No. 3, and the like. As the buffers, there may be mentioned, for example, citric acid, succinic acid, fumaric acid, tartaric acid, ascorbic acid, and salts thereof; glutamic acid, glutamine, glycine, aspartic acid, alanine, arginine, and salts thereof; magnesium oxide, zinc oxide, magnesium hydroxide, phosphoric acid, boric acid, and their salts; and the like. As antioxidants, there may be mentioned, for example, tocopherol, butylhydroxyanisole, ascorbic acid, dibutyl hydroxytoluene, propyl gallate, ascorbate stearate ester, sesamol, oryzanol, quercetin, β-carotene, flavone, and the like. As solubilizers, there may be mentioned, for example, medium chain triglycerides, lecithin, sucrose fatty acid ester, saturated polyglycosylated glyceride (product name: Gelucire), surfactants having an HLB value of 1 to 7, and the like. Further, a substance which does not reduce the transfer of a lipophilic substance which inhibits IL-2 production to blood and does not efficiently deliver the substance to lymph when used alone as the carrier for lymphatic delivery, may be used as the solubilizers, so long as it is contained in an amount which does not affect the reduction of the transfer of the substance to blood nor the lymphatic delivery thereof. As adsorbents, there may be mentioned, for example, silicon dioxide, crystalline cellulose, aluminum silicate, magnesium carbonate, and the like. As absorption inhibitors, there may be mentioned, for example, higher alcohols, fatty acid esters, glutamic acid, and the like. These pharmaceutical fillers may be appropriately used alone or as a mixture of two or more fillers.

The pharmaceutical composition of the present invention may be formulated in various pharmaceutically acceptable forms by conventional methods. For example, a lipophilic substance which inhibits IL-2 production may be dissolved or dispersed in a carrier, and various fillers may be appropriately added to this dispersion to prepare a liquid formulation or a semisolid formulation. Alternatively, the dispersion, or the liquid or semisolid formulation may be used to prepare soft or hard capsules. Alternatively, the dispersion may be mixed with, for example, pharmaceutical fillers such as surfactants or the like and a solvent such as water or the like, and stirred to prepare an emulsion. Alternatively, the dispersion or the emulsion may be solidified by drying, or adsorbing it to an appropriate pharmaceutical filler, to prepare a powder, tablets, or capsules.
Hereinafter, the method of the present invention will be explained.

The present invention relates to a method of inhibiting blood exposure of a lipophilic substance which inhibits IL-2 production and efficiently delivering the substance to lymph in an amount necessary for developing its effects, by administering the substance and a carrier for delivering the substance to lymph.
To develop the pharmacological effects by delivering a lipophilic substance which inhibits IL-2 production to lymph, it is necessary to deliver the substance to lymph in amount medicinally sufficient for treatment or prevention. The present inventors found that when a period of time in which C_{lymph}/IC₅₀ described below is higher than 30 lasts for 1 hour and more, a rate of IL-2 production can be inhibited, even if blood exposure of the substance is inhibited.

The value "C_{lymph}/IC₅₀" is measured and calculated in accordance with test method-8 described below. More particularly, doses of a lipophilic substance which inhibits IL-2 production are varied, and test liquids are forcefully orally administered to transplanted rats using a catheter. In comparison with a rat(s) to which the lipophilic substance which inhibits IL-2 production is not administered, a minimum dose (Xₘᵢₙ) of the substance to significantly prolong the survival of a graft is determined. Lymph concentrations of the lipophilic substance which inhibits IL-2 production (C_{lymph}) following forceful oral administration of test liquids by catheter containing the substance to rats at a dose of Xₘᵢₙ are measured. If lymph concentrations of the lipophilic substance which inhibits IL-2 production are measured at a dose different from Xₘᵢₙ, C_{lymph} may be calculated, assuming that C_{lymph} is proportional to the dose. Further, IC₅₀ (a 50% inhibitory drug concentration in a human mixed lymphocyte reaction) is determined by the above test method-1. A value obtained by dividing C_{lymph} by IC₅₀ is regarded as C_{lymph}/IC₅₀.

The value C_{lymph}/IC₅₀ is a ratio of the concentration of a lipophilic substance which inhibits IL-2 production to develop its effects in lymph in vivo with respect to a concentration of the substance to develop its effect in a mixed lymphocyte reaction in vitro, that is, an index showing how efficiently the effects of the substance are developed in lymph in vivo in comparison with the reaction in vitro. If the effects in vitro and in lymph in vivo are developed at the same efficiency, C_{lymph}/IC₅₀ should be 1. However, taking into consideration that lymph in vivo contains competitors which are adsorbed by a lipophilic substance which inhibits IL-2 production, in comparison with purified and concentrated lymphocytes in vitro, and that lymph circulates around the body, C_{lymph}/IC₅₀ is expected to be more than 1. That is, the concentration of a lipophilic substance which inhibits IL-2 production in lymph in vivo should be higher than IC₅₀ to develop its effects. However, the level and the duration of C_{lymph}/IC₅₀ to develop the effects in vivo have been unknown. The present inventors have conducted intensive studies on this problem, and found that when a lipophilic substance which inhibits IL-2 production and a carrier for delivering the substance to lymph are administered so that a period of time in which C_{lymph}/IC₅₀ is higher than 30 lasts for 1 hour and more, a rate of IL-2 production can be inhibited, even if blood exposure of the substance is inhibited.

By contrast, an accumulated lipophilic substance which inhibits IL-2 production in lymph enhances the risk of development of lymphoma. The present inventors have conducted intensive studies on this problem, and found that when a solubilized powder (a composition consisting of 1 part by weight of tacrolimus, 1 part by weight of hydroxypropyl methylcellulose, 1 part by weight of croscarmellose sodium, and 2 parts by weight of lactose, prepared in accordance with Example 1 of WO 91/19495) contained in Prograf capsules containing tacrolimus was administered, a period of time in which C_{lymph}/IC₅₀ is higher than 30 lasts for less than 4 hours (Experimental Example 3-c described below). Many post-marketing experiences with Prograf capsules have revealed a low risk of development of lymphoma. Therefore, it was found that when a period of time in which C_{lymph}/IC₅₀ is higher than 30 lasts for less than 4 hours, the risk of development of lymphoma due to a lipophilic substance which inhibits IL-2 production transferred to lymph and accumulated in lymph nodes is low.

In patent reference 7 and nonpatent reference 3, a surfactant, such as glycerin fatty acid ester, sugar fatty acid ester, sorbitan fatty acid ester, or the like, is used to disperse cyclosporins into water, and the lymphatic delivery thereof is accomplished. According to an estimate of C_{lymph} in patent reference 7 and nonpatent reference 3 by the present inventors, a period of time in which C_{lymph}/IC₅₀ is higher than 30 lasts for 5 hours or more. It is expected from this estimate that a rate of IL-2 production can be inhibited even though blood exposure of a lipophilic substance which inhibits IL-2 production is inhibited, but the risk of development of lymphoma is higher than that of Prograf capsules (Experimental Example 3-c described below). The increased risk of development of lymphoma is in opposition to a main object of the present invention, alleviation of adverse effects. By contrast, according to the present invention, a period of time in which C_{lymph}/IC₅₀ is higher than 30 lasts from 1 hour to less than 4 hours, and thus, it is expected that a rate of IL-2 production can be inhibited and the risk of development of lymphoma becomes low, even if blood exposure of a lipophilic substance which inhibits IL-2 production is inhibited (Experimental Example 3-c described below).

In the method of the present invention, its embodiments are not particularly limited, so long as desired effects of the present invention can be accomplished.
For example, the method of the present invention can be carried out by administering the pharmaceutical composition of the present invention.

The present invention is **characterized in that** a pharmaceutical composition prepared by selecting, using, and containing a carrier capable of delivering a lipophilic substance which inhibits IL-2 production to lymph is orally administered, and thus, is different from a method of improving oral absorption to enhance bioavailability. Since it was confirmed that the method of the present invention could inhibit blood exposure of a lipophilic substance which inhibits IL-2 production and could develop its pharmacological effect, an immunosuppressive effect, decreased adverse effects such as abnormal renal function or the like are expected, and the method of the present invention exhibits effects different from those of a method of increasing oral absorption.
Hereinafter the use of a carrier of the present invention will be explained.

The present invention relates to a use of specific fats and oils or specific surfactants for inhibiting blood exposure of a lipophilic substance which inhibits IL-2 production, and delivering the substance to lymph in amount necessary for developing its effects, by oral administration.

The present invention is **characterized in that** specific fats and oils or surfactants are used for inhibiting blood exposure of a lipophilic substance which inhibits IL-2 production and delivering the substance to lymph in amount necessary for developing its effects, and thus, is different from a use for enhancing bioavailability. Since it was experimentally confirmed that the use of the present invention could inhibit blood exposure of a lipophilic substance which inhibits IL-2 production and could develop its pharmacological effect, an immunosuppressive effect, decreased adverse effects such as abnormal renal function or the like are expected, and the use of the present invention exhibits effects different from those of a use for increasing oral absorption.

### EXAMPLES

The present invention will now be further illustrated by, but is by no means limited to, the following Examples.

### Example 1 to Example 7

The compositions of the present invention were prepared in accordance with the formulations shown in Table 2, as described below.

**[Table 2]**

| | Ex.1 | Ex.2 | Ex.3 | Ex.4 | Ex.5 | Ex.6 | Ex.7 |
|---|---|---|---|---|---|---|---|
| Tacrolimus | 20 | 20 | 20 | 20 | 20 | 20 | 20 |
| Carrier for lymphatic delivery | | | | | | | |
| Sunflower oil | 20000 | 3000 | - | - | - | - | - |
| Cacao butter | - | - | 3000 | 30000 | - | - | - |
| Medium chain triglycerides | - | - | - | - | 3000 | 30000 | - |
| Monoolein | - | - | - | - | - | - | 30000 |
| Total (mg) | 20020 | 3020 | 3020 | 30020 | 3020 | 30020 | 30020 |

### (Example 1 and Example 2)

Tacrolimus (20 mg) was added to sunflower oil (SIGMA, Sunflower seed oil from Helianthus annuus)(20000 mg or 3000 mg), and the whole was ultrasonicated for 3 hours. After the ultrasonication, tacrolimus was completely dissolved to obtain the composition of the present invention.

### (Example 3 and Example 4)

Cacao butter (Fuji Oil Co., Ltd., Cacao butter 100)(3000 mg or 30000 mg) was heated at approximately 40°C, and tacrolimus (20 mg) was added to the melted cacao butter. The whole was ultrasonicated for 3 hours, and heated at 90°C for 20 minutes. Tacrolimus was completely dissolved to obtain the composition of the present invention.

### (Example 5 and Example 6)

Tacrolimus (20 mg) was added to medium chain triglycerides (The Nisshin OilliO Group, Ltd., O.D.O-H)(3000 mg or 30000 mg), and the whole was ultrasonicated for 3 hours. Tacrolimus was completely dissolved to obtain the composition of the present invention.

### (Example 7)

Monoolein (CRODA, Crossential GMO)(30000 mg) was heated at approximately 40°C, and tacrolimus (20 mg) was added to the melted monoolein. The whole was ultrasonicated for 3 hours, and tacrolimus was completely dissolved to obtain the composition of the present invention.

### Example 8 to Example 11

The compositions of the present invention were prepared in accordance with the formulations shown in Table 3, as described below.

**[Table 3]**

| | Ex.8 | Ex.8-b | Ex.9 | Ex.10 | Ex.11 |
|---|---|---|---|---|---|
| Tacrolimus | 20 | 20 | 20 | 20 | 20 |
| Carrier for lymphatic delivery | | | | | |
| Pluronic L44^{*1} | 20000 | - | - | - | - |
| Pluronic L81^{*1} | - | 20000 | | | |
| Oleic acid | - | - | 30000 | - | - |
| Palmitic acid | - | - | - | 30000 | - |
| Lauric acid | - | - | - | - | 30000 |
| Total (mg) | 20020 | 20020 | 30020 | 30020 | 30020 |

| | | | | | |
|---|---|---|---|---|---|
| *1: Polyoxyethylene polyoxypropylene glycol | | | | | |

### (Examples 8 and 8b and Example 9)

Tacrolimus (20 mg) was added to Pluronic L44 (BASF)(20000 mg), Pluronic L81 (BASF)(20000 mg), or oleic acid (CRODA, CRODACID O-P)(30000 mg), and each mixture was ultrasonicated for 3 hours. Tacrolimus was completely dissolved to obtain the compositions of the present invention.

### (Example 10)

Palmitic acid (Kao Corporation, Lunac P-95)(30000 mg) was heated at approximately 90°C, and tacrolimus (20 mg) was added to the melted palmitic acid and completely dissolved. The whole was cooled to room temperature, and pulverized using a mortar and a pestle to obtain the composition of the present invention.

### (Example 11)

Lauric acid (Kao Corporation, Lunac L-98)(30000 mg) was heated at approximately 90°C, and tacrolimus (20 mg) was added to the melted lauric acid and completely dissolved. The whole was cooled to room temperature, and pulverized using a mortar and a pestle to obtain the composition of the present invention.

### Example 12 to Example 15

The compositions of the present invention were prepared in accordance with the formulations shown in Table 4, as described below.

**[Table 4]**

| | Ex.12 | Ex.13 | Ex.14 | Ex.15 |
|---|---|---|---|---|
| Tacrolimus | 20 | 20 | 20 | 20 |
| Carrier for lymphatic delivery | | | | |
| Medium chain triglycerides | 3000 | 3000 | - | - |
| Pluronic L44^{*1} | - | 3000 | 3000 | - |
| Sunflower oil | 3000 | - | 3000 | - |
| Oleic acid | - | - | - | 2500 |
| Polyglyceryl oleate | - | - | - | 3210 |
| Caprylocaproyl macrogolglyceride | - | - | - | 19290 |
| Water | - | - | - | adequate amount |
| Total (mg) | 6020 | 6020 | 6020 | 100000 |

| | | | | |
|---|---|---|---|---|
| *1: polyoxyethylene polyoxypropylene glycol | | | | |

### (Example 12, Example 13, and Example 14)

Tacrolimus (20 mg) was added to a mixture of medium chain triglycerides (3000 mg) and sunflower oil (3000 mg), a mixture of medium chain triglycerides (3000 mg) and polyoxyethylene polyoxypropylene glycol (3000 mg), or a mixture of polyoxyethylene polyoxypropylene glycol (3000 mg) and sunflower oil (3000 mg), and each mixture was ultrasonicated for 3 hours. Tacrolimus was completely dissolved to obtain the compositions of the present invention.

### (Example 15)

Tacrolimus (30 mg) was added to oleic acid (2500 mg), and ultrasonicated for 3 hours. Tacrolimus was completely dissolved to prepare an oleic acid solution of tacrolimus. Polyglyceryl oleate and caprylocaproyl macrogolglyceride were dissolved in water to prepare an aqueous solution of surfactants. The aqueous solution of surfactants was added dropwise to the oleic acid solution of tacrolimus while stirring, and stirred at room temperature for 8 hours to obtain the composition of the present invention.

### Example 16 to Example 18

The compositions of the present invention were prepared in accordance with the formulations shown in Table 5, as described below.

**[Table 5]**

| | Ex.16 | Ex.17 | Ex.18 |
|---|---|---|---|
| Tacrolimus | 10 | 10 | 10 |
| Carrier for lymphatic delivery | | | |
| Medium chain triglycerides | 500 | 900 | 900 |
| Sunflower oil | 500 | - | - |
| Monoglyceride stearate (MGS-F75MV) | - | 100 | - |
| Monoglyceride stearate (MGS-F50MV) | - | - | 100 |
| Total (mg) | 1010 | 1010 | 1010 |

### (Example 16)

Tacrolimus (10 mg) was added to medium chain triglycerides (500 mg), and the whole was ultrasonicated for 3 hours and completely dissolved. After this solution had cooled to room temperature, sunflower oil (500 mg) was further added thereto and mixed well to obtain the composition of the present invention.

### (Example 17 and Example 18)

Tacrolimus (10 mg) was added to medium chain triglycerides (900 mg), and the whole was ultrasonicated for 3 hours and completely dissolved. Monoglyceride stearate (Nikko Chemicals Co., Ltd., MGS-F75MV)(100 mg) or monoglyceride stearate (Nikko Chemicals Co., Ltd., MGS-F50MV)(100 mg) was further added thereto, and heated at approximately 80°C and completely dissolved. The resulting solution was cooled to room temperature to obtain the composition of the present invention as a semisolid.

### Example 19 and Example 20

The compositions of the present invention were prepared in accordance with the formulations shown in Table 6, as described below.

**[Table 6]**

| | Ex.19 | Ex.20 |
|---|---|---|
| Tacrolimus | 10 | 10 |
| Carrier for lymphatic delivery | | |
| Medium chain triglycerides | 950 | 850 |
| Monoglyceride stearate (MGS-F75MV) | 50 | 150 |
| Total (mg) | 1010 | 1010 |

### (Example 19 and Example 20)

Tacrolimus (10 mg) was added to medium chain triglycerides, and the whole was ultrasonicated for 3 hours and completely dissolved. Monoglyceride stearate (MGS-F75MV) was further added thereto, and heated at approximately 80°C and completely dissolved. The resulting solution was cooled to room temperature to obtain the composition of the present invention as a semisolid.

### Example 21

The composition of the present invention was prepared in accordance with the formulation shown in Table 7, as described below.

**[Table 7]**

| | Ex.21 |
|---|---|
| Tacrolimus | 10 |
| Carrier for lymphatic delivery | |
| Linoleic acid | 900 |
| Monoglyceride stearate (MGS-F75MV) | 100 |
| Total (mg) | 1010 |

### (Example 21)

Tacrolimus (10 mg) was added to linoleic acid (Wako Pure Chemical Industries, Ltd.), and the whole was ultrasonicated for 3 hours and completely dissolved. Monoglyceride stearate (MGS-F75MV) was further added thereto, and heated at approximately 80°C and completely dissolved. The resulting solution was cooled to room temperature to obtain the composition of the present invention as a semisolid.

### Example 22 and Example 23

The compositions of the present invention were prepared in accordance with the formulations shown in Table 8, as described below.

**[Table 8]**

| | Ex.22 | Ex.23 |
|---|---|---|
| Mycophenolate mofetil | 216 | 216 |
| Carrier for lymphatic delivery | | |
| Sunflower oil | 32400 | - |
| Oleic acid | - | 32400 |
| Total (mg) | 32616 | 32616 |

### (Example 22)

Mycophenolate mofetil (216 mg) was added to sunflower oil (32400 mg), and the whole was ultrasonicated for 3 hours and completely dissolved to obtain the composition of the present invention.

### (Example 23)

Mycophenolate mofetil (216 mg) was added to oleic acid (32400 mg), and the whole was stirred and completely dissolved to obtain the composition of the present invention.

The following composition for control was prepared in accordance with Example 1 of WO 91/19495.

| Composition for control | |
|---|---|
| Tacrolimus | 1 |
| Hydroxypropyl methylcellulose | 1 |
| Croscarmellose sodium | 1 |
| Lactose | 2 |
| Total | 5 |

### Comparative Examples 1 to 5

The compositions for comparison were prepared in accordance with the formulations shown in Table 9, as described below.

**[Table 9]**

| | Comp.1 | Comp.2 | Comp.3 | Comp.4 | Comp.5 |
|---|---|---|---|---|---|
| Tacrolimus | 20 | 20 | 20 | 20 | 20 |
| Coconut oil | 30000 | - | - | - | - |
| Caprylic acid | - | 30000 | - | - | - |
| Linolenic acid | - | - | 30000 | - | - |
| Olive oil | - | - | - | 30000 | - |
| Myristic acid | - | - | - | - | 30000 |
| Total (mg) | 30020 | 30020 | 30020 | 30020 | 30020 |

### (Comparative Example 1)

Tacrolimus (20 mg) was added to coconut oil (Nikko Chemicals Co., Ltd., TRIFAT C-24)(30000 mg), and the whole was ultrasonicated for 3 hours and completely dissolved, to obtain a composition for comparison.

### (Comparative Example 2)

Tacrolimus (20 mg) was added to caprylic acid (Kao Corporation, LUNAC 8-98(E))(30000 mg), and the whole was ultrasonicated for 3 hours and completely dissolved, to obtain a composition for comparison.

### (Comparative Example 3)

Tacrolimus (20 mg) was added to linolenic acid (Kanto Chemical Co., Inc.)(30000 mg), and the whole was ultrasonicated for 3 hours and completely dissolved, to obtain a composition for comparison.

### (Comparative Example 4)

Tacrolimus (20 mg) was added to olive oil (CRODA, CLOPURE OL-P)(30000 mg), and the whole was ultrasonicated for 3 hours and completely dissolved, to obtain a composition for comparison.

### (Comparative Example 5)

Myristic acid (Kao Corporation, Lunac MY-98)(30000 mg) was heated at approximately 90°C, and tacrolimus (20 mg) was added to the melted myristic acid and completely dissolved. The whole was cooled to room temperature, and pulverized using a mortar and a pestle to obtain a component for comparison.

### Experimental Example 1 (Pharmacokinetics/pharmacodynamics evaluations of tacrolimus in rats)

The formulations prepared in Examples 1 to 3, 5 to 15, and 17 to 21, the composition for control, and the formulations prepared in Comparative Examples 1 to 5 were orally administered to rats (male Lewis rat, 6 weeks), and blood exposure of tacrolimus (blood AUC_{blood}) and its pharmacological effect (IL-2 production rate) were evaluated.

The dose of tacrolimus was 1 mg/kg, 2 mg/kg, or 3 mg/kg. Tacrolimus was forcefully orally administered using a catheter to rats which had been fasted for 12 hours or more. The formulations prepared in Examples 1, 2, 5, 6, 8, 9, and 12 to 15 and Comparative Examples 1 to 4 were administered as solutions, without further being processed before the administration. The formulations prepared in Examples 17 to 21 were administered as semisolids, without further being processed. The formulations prepared in Examples 3 and 7 were heated to approximately 40°C before the administration. The formulations prepared in Examples 10 and 11 and Comparative Example 5 were suspended at a concentration of 0.5% by weight, in a 0.5% by weight methylcellulose (Shin-Etsu Chemical Co., Ltd., SM-400) aqueous solution, before the administration. The composition for control was suspended in water at a concentration of 5 mg/mL, before the administration.

A blood AUC_{blood} was measured and calculated in accordance with test method-4. Rats were fasted until a blood collection following the administration of test liquids, but were allowed to freely take water. Blood samples were collected after 1, 4, 8, 12, and 19 hours from the administration. Whole blood concentrations of tacrolimus were measured by LC-MS/MS (TSQ, ThermoQuest), and areas under the blood concentration versus time curve (blood AUC_{blood}) were calculated by the trapezoidal rule.

A rate of IL-2 production was measured and calculated in accordance with test method-3. Rats were fasted until a blood collection following the administration of test liquids, but were allowed to freely take water. Concanavalin A (Funakoshi Co., Ltd, Lot: S0619) was added to physiological saline at a concentration of 1 mg/mL, and the whole was stirred for 5 minutes and passed through a filter of 0.45 µm (NALGENE, SFCA Filter Unit) to obtain a filtrate (hereinafter referred to as concanavalin A solution). After 16 hours had passed from the administration of test liquids to rats, the concanavalin A solution (within 2 hours of the preparation) was rapidly intravenously administered to the rats, at a dose of 15 mg/kg as concanavalin A. Blood samples were collected after 3 hours from the administration of the concanavalin A solution, and centrifuged to obtain plasma samples. An ELISA kit (BioSource International, Inc., Rat IL-2 ELISA kit) was used to measure plasma concentrations of IL-2. Similarly, the concanavalin A solution was rapidly intravenously administered to rats to which no test liquid was administered (hereinafter referred to as the untreated group); blood samples were collected after 3 hours from the administration, and centrifuged to obtain plasma samples; and plasma concentrations of IL-2 were measured by the ELISA kit (BioSource International, Inc., Rat IL-2 ELISA kit). The percentage (%) of a plasma concentration of IL-2 in a rat to which a test liquid was administered with respect to that in the untreated group was regarded as a rate of IL-2 production.
Blood AUC_{blood} and the rate of IL-2 production after the administration of each formulation are shown in Table 10.

**[Table 10]**

| Formulation | Tacrolimus dose (mg/kg) | Blood AUC_{blood} (ng·h/mL) | Average rate of IL-2 production (n=3) (%) |
|---|---|---|---|
| Control | 3 | 130 | 3 |
| Ex.1 | 3 | 29^{b} | 6 |
| Ex.2 | 2 | 60 | 10 |
| Ex.3 | 2 | 41 | 10 |
| Ex.5 | 2 | 75 | 7 |
| Ex.6 | 2 | 29 | 16 |
| Ex.7 | 3 | 11 | 18 |
| Ex.8 | 1 | 35 | 25 |
| Ex.9 | 2 | 27 | 7 |
| Ex.10 | 2 | 43^{a} | 16 |
| Ex.11 | 2 | 39^{a} | 14 |
| Ex.12 | 2 | 87 | 13 |
| Ex.13 | 2 | 66 | 9 |
| Ex.14 | 2 | 70 | 8 |
| Ex.15 | 2 | 26^{b} | 15 |
| Ex.17 | 3 | 63 | 13 |
| Ex.18 | 3 | 79 | 8^{d} |
| Ex.19 | 3 | 79 | 10^{e} |
| Ex.20 | 3 | 78 | 8 |
| Comp.1 | 2 | 15 | 51 |
| Comp.2 | 2 | 139 | 7 |
| Comp.3 | 2 | 40 | 104 |
| Comp.4 | 2 | N.A.^{c} | 48 |
| Comp.5 | 2 | N.A.^{c} | 53 |

| | | | |
|---|---|---|---|
| Blood AUC_{blood} was calculated from average whole blood concentration at each blood collection point (n=3). Notes) ^{a}; Blood AUC_{blood} calculated from whole blood concentrations of tacrolimus after 1, 4, 8, and 12 hours from administration. ^{b}; Blood AUC_{blood} calculated from whole blood concentrations of tacrolimus after 1, 4, and 8 hours from administration. ^{c}; Blood AUC was not calculated. ^{d}; Average (n=6). ^{e}; Average (n=8). | | | |

Relative blood AUC_{blood} to that of the composition for control and the rate of IL-2 production after the administration of the formulation prepared in Example 21 are shown in Table 11.

**[Table 11]**

| Formulation | Tacrolimus dose (mg/kg) | Relative blood AUC_{blood} (%) | Average rate of IL-2 production (n=3) (%) |
|---|---|---|---|
| Control | 3 | 100 | 3 |
| Ex.21 | 3 | 48 | 10^{a} |

| | | | |
|---|---|---|---|
| Blood AUC_{blood} was calculated from average whole blood concentration at each blood collection point (n=3) ^{a}; n=1. | | | |

The formulations prepared in Examples 1 to 3, 5 to 15, and 17 to 21 inhibited IL-2 production and exhibited pharmaceutical effects similar to those of the composition for control, but blood AUC_{blood} was reduced to 67% or less, relative to the composition for control. This result indicated that blood exposure of tacrolimus was decreased. By contrast, some of the formulations prepared in Comparative Examples 1 to 5 inhibited IL-2 production similarly to the composition for control, but did not exhibit decreased blood AUC_{blood}; or some thereof exhibited decreased blood AUC_{blood} in comparison with the composition for control, but did not inhibit IL-2 production sufficiently. These results reveal that the pharmaceutical composition of the present invention inhibits blood exposure of tacrolimus, and thereby reduces adverse effects due to continuous high blood exposure and develops desired pharmacological effects of tacrolimus.

### Experimental Example 2

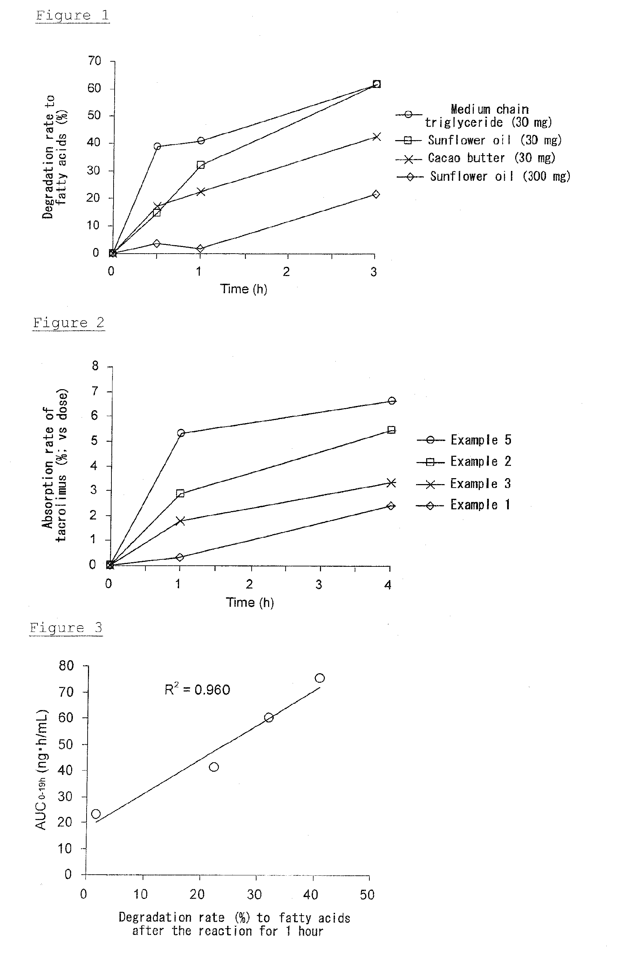

A degradation rate to fatty acids was measured and calculated in accordance with test method-6. Sodium taurocholate was dissolved in a phosphate buffer (pH6.8) (Japanese Pharmacopeia, Thirteenth Edition, a disintegration test, the second fluid) at a concentration of 50 mmol/L. Lipase (SIGMA, Lipase from porcine pancreas, 329 U/mg) was added thereto at a concentration of 375 U/mL, and stirred for 2 hours to prepare simulated intestinal fluid. As samples before a reaction at approximately 28°C while shaking (YAMATO SCIENTIFIC CO., LTD., maximum speed), 30 mg or 300 mg of each base was added to 6.0 mL of the simulated intestinal fluid. As samples after the reaction, these samples were shaken at approximately 28°C for 0.5, 1, or 3 hours. A kit for measuring nonesterified fatty acids (Wako Pure Chemical Industries, Ltd., NEFA C Test Wako) was used to measure concentrations of nonesterified fatty acids contained in the samples before the reaction and the samples after the reaction. Further, fatty acid compositions described in the Handbook of Oil Chemistry (Revised 3rd ed., Japan Oil Chemists' Society, Maruzen Co., Ltd.) were used to calculate theoretical concentrations of fatty acids generated when each base is completely degraded. The percentage (%) of a difference between a concentration of nonesterified fatty acids contained in a sample after the reaction and a concentration of nonesterified fatty acids contained in a sample before the reaction with respect to a theoretical concentration of fatty acids was regarded as the degradation rate to fatty acids. With respect to samples in which 30 mg of medium chain triglycerides, sunflower oil, or cacao butter was added, and a sample in which 300 mg of sunflower oil was added, cumulative degradation rates to fatty acids are shown in Figure 1.

As shown in Figure 1, degradation rates to fatty acids varied according to the kind of base, and the order of the degradation rates was as follows:
cacao butter < sunflower oil < medium chain triglycerides.
Further, degradation rates to fatty acids varied according to the amount of base, and it was found that an increased amount of a base reduces the degradation rate.

Next, blood concentrations of tacrolimus following administration of the compositions prepared in Examples 1, 2, 3, and 5, obtained in Experimental Example 1, and blood concentrations following intravenous injection of tacrolimus were used to carry out a deconvolution analysis. The tacrolimus for the intravenous injection was dissolved in PEG400 (Kanto Chemical Co., Ltd.) at a concentration of 0.1 mg/mL, and intravenously administered to rats (male Lewis rat, 6 weeks) at a dose of 0.1 mg/kg as tacrolimus. The obtained cumulative absorption rates of tacrolimus following oral administration of the compositions of the present invention prepared in Examples 1, 2, 3, and 5 are shown in Figure 2.

As shown in Figure 2, the absorption rates varied according to the kind of base, and the order of the absorption rates was as follows:
Example 3 (cacao butter) < Example 2 (sunflower oil) < Example 5 (medium chain triglycerides).
Further the comparison of Example 2 (sunflower oil) with Example 1 (sunflower oil) revealed that an increased amount of base administered (Example 1) reduces the absorption rate. From the comparison between Figures 1 and 2, the degradation rate of a base is considered to be relevant to the absorption rate of tacrolimus. The correlation between the degradation rates to fatty acids after the reaction for 1 hour, obtained in Experimental Example 2, and the blood AUC₀₋₁₉ₕ of tacrolimus following administration of the formulations prepared in Examples 1, 2, 3, and 5, obtained in Experimental Example 1, is shown in Figure 3.

As shown in Figure 3, there was a good correlation between the degradation rates to fatty acids after the reaction for 1 hour obtained in Experimental Example 2 and the blood AUC₀₋₁₉ₕ of tacrolimus.
These results suggested that a formulation with a low degradation rate to fatty acids after the reaction for 1 hour (determined by the procedure described in Experimental Example 2) is suitable to inhibit blood exposure of tacrolimus and alleviate adverse effects of tacrolimus.

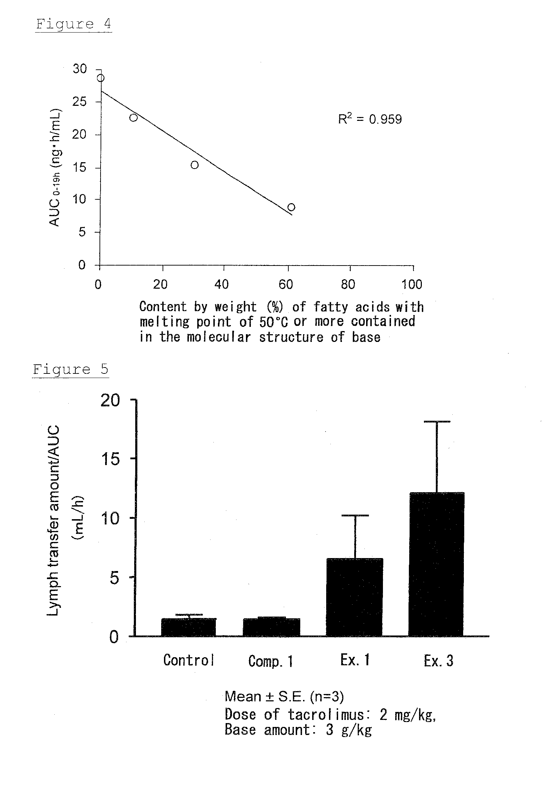

Further, a correlation between chemical structures of bases and blood AUC_{blood} of tacrolimus were examined, and there was a good correlation between the content by weight of fatty acids having a melting point of 50°C or more contained in the molecular structure of a base and blood AUC_{blood} of tacrolimus following administration of the formulations prepared in Examples 1, 4, and 6 and Comparative Example 1 (Figure 4). This result indicated that a base including an increased content by weight of fatty acids having a melting point of 50°C or more contained in the molecular structure is suitable to inhibit blood exposure of tacrolimus and alleviate adverse effects of tacrolimus.

### Experimental Example 3-a (Evaluation of lymphatic transfer) of tacrolimus in rats)

The formulations prepared in Examples 1 and 3, the composition for control, and the formulation prepared in Comparative Example 1 were used to evaluate the lymphatic transfer of tacrolimus after oral administration of each sample to rats (male Lewis rat, 300-350 g).

The dose of tacrolimus was 2 mg/kg. The formulations prepared in Example 1 and Comparative Example 1 were administered as solutions, without being further processed before the administration. The formulation prepared in Example 3 was heated to approximately 40°C before the administration. The composition for control was suspended in water at a concentration of 5 mg/mL, before the administration.

An amount of tacrolimus delivered to lymph was measured and calculated. The thoracic lymph duct was cannulated in rats, and it was confirmed that lymph could be stably collected. Each test liquid was forcefully orally administered to the rats using a catheter. The rats were fasted until lymph collections following the administration of test liquids were completed, but were allowed to freely take water. Concentrations of tacrolimus contained in lymph samples collected during predetermined periods of time (0-1, 1-2, 2-3, 3-4, 4-6, and 6-24 hours after the administration) were measured by LC-MS/MS. Each lymph concentration of tacrolimus was multiplied by the volume of lymph collected during each period of time, and the resulting values were totalized to calculate a lymph transfer amount.

A blood AUC_{blood} was measured and calculated in accordance with test method-4. Rats were fasted until a blood collection following the administration of test liquids, but were allowed to freely take water. Blood samples were collected after 1, 4, 8, 12, and 19 hours from the administration. Whole blood concentrations of tacrolimus were measured by LC-MS/MS, and areas under the blood concentration versus time curve (blood AUC_{blood}) were calculated by the trapezoidal rule.

The lymphatic transfer of each composition was calculated as a value obtained by dividing a lymph transfer amount of tacrolimus by a blood AUC_{blood}. The lymphatic transfer of each composition is shown in Figure 5.
As shown in Figure 5, the formulations prepared in Examples 1 and 3 elevated a lymph transfer amount per unit blood AUC_{blood}, relative to the composition for control, and exhibited a high lymphatic transfer. By contrast, the formulation prepared in Comparative Example 1 exhibited the same lymphatic transfer as that of the composition for control. These results revealed that the pharmaceutical composition of the present invention efficiently delivers tacrolimus to lymph, and thereby alleviates adverse effects due to continuous high blood exposure and develops desired pharmacological effects of tacrolimus.

### Experimental Example 3-b (Comparison between blood exposure of tacrolimus and lymph transfer amount in rats)

The formulations prepared in Examples 1 and 3, the composition for control, and the formulation prepared in Comparative Example 1 were orally administered to rats (male Lewis rat, 300-350 g), and the blood exposure of tacrolimus was compared to the lymph transfer amount.

The dose of tacrolimus was 2 mg/kg. The formulations prepared in Example 1 and Comparative Example 1 were administered as solutions, without being further processed before the administration. The formulation prepared in Example 3 was heated to approximately 40°C before the administration. The composition for control was suspended in water at a concentration of 5 mg/mL, before the administration.

A blood AUC_{blood} was measured and calculated in accordance with test method-4. Rats were fasted until a blood collection following the administration of test liquids, but were allowed to freely take water. Blood samples were collected after 1, 4, 8, 12, and 19 hours from the administration. Whole blood concentrations of tacrolimus were measured by LC-MS/MS, and areas under the blood concentration versus time curve (blood AUC_{blood}) were calculated by the trapezoidal rule.

A lymph AUC_{lymph} was measured and calculated in accordance with test method-2. The thoracic lymph duct was cannulated in rats, and it was confirmed that lymph could be stably collected. Each test liquid was forcefully orally administered to the rats using a catheter. The rats were fasted until lymph collections following the administration of test liquids were completed, but were allowed to freely take water. Concentrations of tacrolimus contained in lymph samples collected during predetermined periods of time (0-1, 1-2, 2-3, 3-4, 4-6, and 6-24 hours after the administration) were measured by LC-MS/MS. Areas under the lymph concentration versus time curve (lymph AUC_{lymph}) were calculated by the trapezoidal rule, as a drug concentration at the midpoint of the period of lymph collection.
The blood AUC_{blood} and the lymph AUC_{lymh} (average of three calculated values) following the administration of each composition are shown in Table 12.

**[Table 12]**

| | Blood AUC_{blood} (ng*h/ml) | Lymph AUC_{lymph} (ng*h/mL) |
|---|---|---|
| Control | 65.3 | 83.5 |
| Comp.1 | 15.3 | 13.5 |
| Ex.1 | 22.5 | 137.3 |
| Ex.3 | 8.8 | 74.6 |

As shown in Table 12, the formulations prepared in Comparative Example 1 and Examples 1 and 3 reduced blood AUC_{blood}, relative to the composition for control. The formulations prepared in Examples 1 and 3 exhibited equivalent lymph AUC_{lymph} to that of the composition for control, and were expected to develop the same effects as that of the composition for control. The formulation prepared in Comparative Example 1 exhibited decreased lymph AUC_{lymph}, and thus, decreased effects were predicted. These results suggested that the pharmaceutical composition of the present invention reduces adverse effects due to continuous high blood exposure and develops desired pharmacological effects of tacrolimus.

### Experimental Example 3-c (Pharmacodynamics evaluations of tacrolimus and concentration thereof in lymph cells in rats)

In accordance with test method-3 and test method-7, each preparation prepared in Examples 16 and 17 and the composition for control were orally administered to rats (male Lewis rat, 6 weeks), and the concentration of tacrolimus contained in 10⁷ lymph cells and the pharmacological effect (rate of IL-2 production) were evaluated for each. The concentrations thereof in lymph cells after the administration of each formulation and the rates of IL-2 production are shown in Table 13.

**[Table 13]**

| | Concentration in lymph cells (ng/10⁷ cells) | Rate of IL-2 production (%) |
|---|---|---|
| Control | 2.3 | 6 |
| Ex.16 | 2.9 | 8^{a} |
| Ex.17 | 3.7 | 13 |

| | | |
|---|---|---|
| Average (n=3) ^{a};Average (n=6) | | |

The formulations prepared in Examples 16 and 17, as well as the composition for control, inhibited IL-2 production, and showed the same concentrations of tacrolimus contained in lymph cells as that in the control. These results show that a concentration in lymph cells can be used as an index for the inhibition of IL-2 production.

### Experimental Example 3-d (Comparison between blood exposure of mycophenolic acid and concentration thereof in lymph cells after administration of mycophenolate mofetil in rats)

Mycophenolate mofetil which is orally administered is rapidly metabolized to mycophenolic acid as an active form. Each formulation prepared in Examples 22 and 23 and the composition for control were orally administered to rats (male Lewis rat, 6 weeks), and the blood exposure (plasma AUCₚₗₐₛₘₐ) of mycophenolic acid and the pharmacological effect (concentration in lymph cells) were evaluated for each.

The dose of mycophenolate mofetil was 20 mg/kg. Mycophenolate mofetil was forcefully orally administered using a catheter to rats which had been fasted for 12 hours or more. The formulations prepared in Examples 22 and 23 were administered as solutions, without being further processed before the administration. As a control composition, mycophenolate mofetil was suspended at a concentration of 4 mg/mL in a 0.5% by weight methylcellulose (Shin-Etsu Chemical Co., Ltd., SM-400) aqueous solution, before administration.

A plasma AUCₚₗₐₛₘₐ was measured and calculated in accordance with test method-4. Rats were fasted until a blood collection following the administration of test liquids, but were allowed to freely take water. Blood samples were collected after 0.5, 1, and 2 hours from the administration, and centrifuged to obtain plasma samples. Plasma concentrations of mycophenolic acid were measured by HPLC (Shimadzu), and areas under the plasma concentration versus time curve (plasma AUCₚₗₐₛₘₐ) were calculated by the trapezoidal rule.

The concentration of mycophenolic acid in lymph cells was measured and calculated in accordance with test method-7. Rats were fasted until a blood collection following the administration of test liquids, but were allowed to freely take water. Blood samples were collected after 2 hours from the administration. Lymph cells are isolated utilizing a Ficcoll-conray solution (Lymphosepar II, Immune-Biological Laboratories Co., Ltd.) by the density gradient method of sucrose. Each concentration of mycophenolic acid contained in 10⁷ lymph cells is measured by HPLC (Shimadzu).
The plasma AUCₚₗₐₛₘₐ and the concentration thereof in lymph cells after the administration of each formulation are shown in Table 14.

**[Table 14]**

| Formulation | Mycophenolate mofetil dose (mg/kg) | Plasma AUCₚₗₐₛₘₐ (µg·h/mL) | Average concentration in lymph cells (n=3) (ng/10⁷ cell) |
|---|---|---|---|
| Control | 20 | 17^{a} | 119 |
| Ex.22 | 20 | 7^{a} | 137 |
| Ex.23 | 20 | 2^{a} | 110 |

| | | | |
|---|---|---|---|
| Plasma AUCₚₗₐₛₘₐ was calculated from average plasma concentration at each plasma collection point (n=3). Note) ^{a}; Plasma AUCₚₗₐₛₘₐ calculated from plasma concentrations of mycophenolic acid after 0.5, 1, and 2 hours from administration. | | | |

The formulations prepared in Examples 22 and 23 exhibited similar concentrations of mycophenolic acid in lymph cells and pharmaceutical effects to those of the composition for control, but plasma AUCₚₗₐₛₘₐ was reduced 39% or less, relative to the composition for control. This result indicated that blood exposure of mycophenolic acid was decreased. Further, when a suspension of mycophenolate mofetil, as a control, was orally administered to rats at a dose of 20 mg/kg, it was shown that the survival of a graft was significantly prolonged in a rat heart graft model [Transplant 64, 1695, 1997, Celcept (registered trademark) Drug Interview Form, February, 2008 (12th revised edition)]. This suggested that the pharmaceutical composition of the present invention reduces adverse effects due to continuous high blood exposure and develops desired pharmacological effects of mycophenolate mofetil.

### Experimental Example 3-e (Comparison of C_{lymph}/IC₅₀ of tacrolimus and cyclosporin in rats)

The formulations prepared in Examples 1 and 3, the composition for control, and the formulation prepared in Comparative Example 1 were orally administered to rats (male Lewis rat, 300-350 g), and C_{lymph}/IC₅₀ values were compared.

The dose of tacrolimus was 2 mg/kg. The formulations prepared in Example 1 and Comparative Example 1 were administered as solutions, without being further processed before the administration. The formulation prepared in Example 3 was heated to approximately 40°C before the administration. The composition for control was suspended in water at a concentration of 5 mg/mL, before the administration.

C_{lymph}/IC₅₀ was measured and calculated in accordance with test method-8. When the dose of tacrolimus was varied and suspensions of a solubilized powder contained in Prograf capsules containing tacrolimus were forcefully orally administered using a catheter to transplant rats, a minimum dose (Xₘᵢₙ) of tacrolimus to significantly prolong the survival of a graft was 1 mg/kg, in comparison with the rats to which tacrolimus was not administered (Transplantation, 44, 734-738, 1987). As apparent from the results of Experimental Example 4 described below, the dose of the suspensions of a solubilized powder contained in Prograf capsules containing tacrolimus are the same as the minimum dose of tacrolimus to significantly prolong the survival of a graft in Example 1. Therefore, a lymph concentration of tacrolimus (C_{lymph}) following administration of tacrolimus at a dose of Xₘᵢₙ was calculated by dividing a lymph concentration of tacrolimus following administration of tacrolimus at a dose of 2 mg/kg by 2. Further, IC₅₀ (a 50% inhibitory drug concentration in a human mixed lymphocyte reaction) of tacrolimus was 0.18 ng/mL (Transplant Proceedings, No 5, Suppl 6, 36-39, 1987). The value obtained by dividing C_{lymph} by IC₅₀ is regarded as C_{lymph}/IC₅₀, and the result (average of three calculated values) is shown in Table 15.

**[Table 15]**

| Time from adm. (h) | Control | Comp.1 | Ex.1 | Ex.3 |
|---|---|---|---|---|
| 0.5 | 21 | 1 | 164 | 166 |
| 1.5 | 67 | 3 | 176 | 45 |
| 2.5 | 35 | 4 | 16 | 7 |
| 3.5 | 23 | 3 | 10 | 4 |
| 5 | 12 | 3 | 7 | 4 |
| 15 | 3 | 1 | 2 | 1 |

Data for lymphatic delivery of cyclosporin A described in patent reference 7 and nonpatent reference 3 were used to calculate C_{lymph}/IC₅₀ in accordance with test method-8. In experiments for collecting lymph samples of Referential Example of patent reference 7 and nonpatent reference 3, 3.5 mg of cyclosporin A was administered to rats (350 to 450 g), and a dose of cyclosporin A was approximately 8.75 mg/kg. Further, when doses of cyclosporin A were varied, and the contents of Sandimmun capsules containing cyclosporin A were administered to transplanted rats were forcefully orally administered using a catheter, a minimum dose of cyclosporin A to significantly prolong the survival of a graft was 10 mg/kg, in comparison with the rats to which cyclosporin A was not administered (Transplantation, 44, 734-738, 1987). Furthermore, according to the result of skin grafting in rats described in nonpatent reference 3, administration of the invention (HCO-60 formulation) described in patent reference 7 at a dose of 1 mg/kg developed the same effects as that of Comparative Example (sesame oil formulation, 7 to 15 mg/kg) of patent reference 7, and Xₘᵢₙ of the composition described in patent reference 7 and nonpatent reference 3 is considered 1 mg/kg. Lymph concentration (C_{lymph}) of cyclosporin A following administration of cyclosporin A at a dose of Xₘᵢₙ was calculated by dividing lymph concentration of cyclosporin A at a dose of 8.75 mg/kg by 8.75. IC₅₀ (a 50% inhibitory drug concentration in a human mixed lymphocyte reaction) of cyclosporin was 16.8 µg/mL (Transplant Proceedings, No 5, Suppl 6, 36-39, 1987). The value obtained by dividing C_{lymph} by IC₅₀ is regarded as C_{lymph/}IC₅₀ (Table 16 and Table 17)_{.}

**[Table 16]**

| Time from adm. (h) | Ex.1 in patent reference 7 | Ex.2 in Patent reference 7 | Ex.3 in Patent reference 7 | Comp.1 in Patent reference 7 | Comp.2 in Patent reference 7 | Comp.3 in Patent reference 7 |
|---|---|---|---|---|---|---|
| 0.5 | 19 | 81 | 34 | 7 | 7 | 4 |
| 1.5 | 43 | 543 | 713 | 14 | 10 | 3 |
| 2.5 | 107 | 353 | 747 | 12 | 24 | 3 |
| 3.5 | 109 | 224 | 319 | 12 | 12 | 3 |
| 4.5 | 81 | 183 | 143 | 11 | 10 | 3 |
| 5.5 | 88 | 122 | 81 | 10 | 7 | 3 |

**[Table 17]**

| Time from adm. (h) | Formulation HCO-60 nonpatent reference 3 | Formulation sugar ester solution in nonpatent reference 3 | Formulation mixed micelle solution in nonpatent reference 3 | Formulation sesame oil in nonpatent reference 3 | Formulation linoleic acid in nonpatent reference 3 |
|---|---|---|---|---|---|
| 0.5 | 102 | 54 | 14 | 7 | 0 |
| 1.5 | 387 | 122 | 41 | 14 | 0 |
| 2.5 | 380 | 272 | 109 | 20 | 0 |
| 3.5 | 204 | 305 | 115 | 14 | 0 |
| 4.5 | 176 | 258 | 75 | 14 | 0 |
| 5.5 | 136 | 149 | 95 | 7 | 0 |

As shown in Tables 15 to 17, according to the method of the present invention, and the methods described in patent reference 7 and nonpatent reference 3, a period of time in which C_{lymph}/IC₅₀ is higher than 30 lasts for 1 hour and more, and thus, it was found that the inhibition of a rate of IL-2 production is expected, even if blood exposure of a lipophilic substance which inhibits IL-2 production is inhibited. However, in the methods described in patent reference 7 and nonpatent reference 3, it is considered that the risk of development of lymphoma is higher than that of Prograf capsules, because a period of time in which C_{lymph}/IC₅₀ is higher than 30 lasts for 5 hours or more. The increased risk of development of lymphoma is opposed to a main object of the present invention, alleviation of adverse effects. By contrast, according to the method of the present invention, a period of time in which C_{lymph}/IC₅₀ is higher than 30 lasts from 1 hour to less than 4 hours, and thus, it is expected that a rate of IL-2 production can be inhibited and the risk of development of lymphoma becomes low, even if blood exposure of the lipophilic substance which inhibits IL-2 production is inhibited.

### Experimental Example 4 (Effects on abdominal heterotopic heart grafted in rats)

Transplant model animals were used to evaluate pharmacological effects according to the survival time of the transplanted organ following the administration of the composition for control, or the formulations prepared in Examples 1, 16, and 17. The heart was taken from each rat (male Lewis rat, 6 weeks), and transplanted into the abdomen of another rat. The dose of tacrolimus was 3 mg/kg. The formulation prepared in Example 1 was administered as a solution, without being further processed before the administration. The composition for control was suspended in water at a concentration of 5 mg/mL, before the administration. Each test liquid was forcefully orally administered using a catheter. Only water was orally administered to a placebo group. The rats were not fasted and were allowed to freely take water. Each formulation was repeatedly orally administered to the transplant rats once a day for 14 days following the transplantation.

The formulations prepared in Examples 1, 16, and 17, and the composition for control prolonged the survival of transplanted organ, in comparison with the placebo group. Further, the formulations prepared in Examples 1, 16, and 17 showed the same prolonged survival of transplanted organ as the composition for control.

### Experimental Example 5 (Nonfasting pharmacokinetic test)

Under the same conditions for administration to the transplanted model animals as shown in Experimental Example 4, the composition for control and the formulation prepared in Example 1 were repeatedly orally administered once a day for 14 days, and blood concentrations were monitored. Rats were not fasted and were allowed to freely take water. Blood samples were collected after 2, 4, 8, 12, 18, and 24 hours from the administration at Day 14, and whole blood concentrations of tacrolimus were measured by LC-MS/MS. From the measured values, maximum blood concentrations (Cmax) and areas under the blood concentration versus time curve (blood AUC₀₋₂₄ₕ) were calculated.
Cmax and blood AUC₀₋₂₄ₕ (n=3) after the administration of each formulation are shown in Table 18.

**[Table 18]**

| | Cmax (ng/ml) | Blood AUC₀₋₂₄ₕ (ng*h/mL) |
|---|---|---|
| Control | 46.1 | 203.3 |
| Ex.1 | 7.2 | 66.9 |

Cmax and blood AUC₀₋₂₄ₕ of the formulation prepared in Example 1 were reduced 16% and 33%, respectively, relative to the composition for control.

### Experimental Example 6 (pharmacokinetic test of tacrolimus in cynomolgus monkeys)

The formulations prepared in Examples 16 and 17, and the composition for control were orally administered to cynomolgus monkeys (male, 3-5 years, 2.8-3.8 kg), and blood exposure (blood AUC_{blood}) of tacrolimus were evaluated.

The dose of tacrolimus was 1 mg/body. Capsules for administration were forcefully orally administered, together with 10 mL of water, to cynomolgus monkeys which had been fasted for 16 hours or more. These capsules for administration were prepared by filling No. 4 gelatin capsules (Capsugel Japan Inc.) with a solution (Example 16) or a semisolid (Example 17), or No. 5 gelatin capsules with a mixture of 5 parts by weight of the composition for control, 59.35 parts by weight of lactose, and 0.65 parts by weight of stearic acid.

A blood AUC_{blood} was measured and calculated in accordance with test method-5. The monkeys were fasted until the blood collection after 24 hours from the administration of the capsules was completed, but were allowed to freely take water. Blood samples were collected before the administration, and after 0.5, 1, 2, 4, 6, 8, 12, 24, and 48 hours from the administration. Whole blood concentrations of tacrolimus were measured by LC-MS/MS, and areas under the blood concentration versus time curve (blood AUC_{blood}) were calculated by the trapezoidal rule.
Cmax and blood AUC₀₋₄₈ₕ (n=5) after the administration of each formulation are shown in Table 19.

**[Table 19]**

| | Cmax (ng/ml) | Blood AUC₀₋₄₈ₕ (ng*h/mL) |
|---|---|---|
| Control | 26±21 | 188±160 |
| Ex.16 | 10±7 | 85±42 |
| Ex.17 | 9±6 | 50±27 |

| | | |
|---|---|---|
| Mean±S.D. (n=5) | | |

Average Cmax of the formulations prepared in Examples 16 and 17 were reduced to 38% and 35%, respectively, relative to the composition for control. Similarly, average blood AUC₀₋₄₈ₕ thereof were reduced to 45% and 27%, respectively.
These results revealed that the pharmaceutical composition of the present invention inhibits blood exposure of tacrolimus, and thereby alleviates adverse effects due to continuous high blood exposure and develops desired pharmacological effects of tacrolimus.

### INDUSTRIAL APPLICABILITY

It has been confirmed that the pharmaceutical composition of the present invention inhibits blood exposure of a lipophilic substance which inhibits IL-2 production, and develops its pharmacological effect, an immunosuppressive effect, and thus, alleviation of adverse effects such as abnormal renal function or the like is expected. The present invention exhibits effects different from those of a technology of enhancing oral absorption.
As above, the present invention was explained with reference to particular embodiments, but modifications and improvements obvious to those skilled in the art are included in the scope of the present invention.

## Claims

1. A pharmaceutical composition comprising a lipophilic substance which inhibits IL-2 production, and a carrier capable of inhibiting blood exposure of the substance and delivering the substance to lymph.

2. The pharmaceutical composition according to claim 1, wherein the lipophilic substance which inhibits IL-2 production has a LogP value of 2 or more and an IC₅₀ (a 50% inhibitory drug concentration in a human mixed lymphocyte reaction) of 1 µmol/L or less.

3. The pharmaceutical composition according to claim 1 or 2, wherein the lipophilic substance which inhibits IL-2 production is one or two or more substances selected from the group consisting of tacrolimus, cyclosporin, rapamycin, mycophenolic acid, and mycophenolate mofetil.

4. The pharmaceutical composition according to any one of claims 1 to 3, wherein the carrier capable of inhibiting exposure to the substance in blood and delivering the substance to lymph is one or two or more substances selected from the group consisting of (i) a mono-, di-, or tri-glyceride, or a mixture thereof, (ii) a fatty acid having 12 to 18 carbon atoms, or a mixture thereof, (iii) a surfactant having an HLB value of 10 to 18 and containing polyethylene glycol having a weight average molecular weight of 200 to 5000 in its molecular structure, and (iv) a higher alcohol having 12 to 18 carbon atoms.

5. The pharmaceutical composition according to claim 4, wherein (i) is one or two or more substances selected from the group consisting of rapeseed oil, castor oil, sunflower oil, cacao butter, medium chain triglycerides, monoolein, diolein, triolein, monoglyceride stearate, diglyceride stearate, triglyceride stearate, monoglyceride palmitate, diglyceride palmitate, triglyceride palmitate, monoglyceride myristate, diglyceride myristate, triglyceride myristate, monoglyceride arachidate, diglyceride arachidate, triglyceride arachidate, monoglyceride behenate, diglyceride behenate, and triglyceride behenate.

6. The pharmaceutical composition according to claim 4, wherein (ii) is one or two or more substances selected from the group consisting of linoleic acid, stearic acid, oleic acid, lauric acid, and palmitic acid.

7. The pharmaceutical composition according to claim 4, wherein (iii) is one or two or more substances selected from the group consisting of polyoxyethylene polyoxypropylene glycol, polyoxyethylene fatty acid monoester, saturated polyglycosylated glyceride, D-α-tocopheryl polyethylene glycol 1000 succinate, and polyoxyethylene hydrogenated castor oil.

8. The pharmaceutical composition according to claim 4, wherein (iv) is one or two or more substances selected from the group consisting of stearyl alcohol, myristyl alcohol, and cetyl alcohol.

9. The pharmaceutical composition according to any one of claims 1 to 8, wherein the content of the carrier is 0.1 to 20,000 parts by weight with respect to 1 part by weight of the lipophilic substance which inhibits IL-2 production.

10. The pharmaceutical composition according to claim 1, which is for oral administration.

11. The pharmaceutical composition according to claim 1, which is for lymphatic delivery.

12. A method of delivering a lipophilic substance which inhibits IL-2 production to lymph, comprising administering to a subject a carrier capable of inhibiting blood exposure of the substance and delivering the substance to lymph.

13. The method according to claim 12, wherein a period of time in which C_{lymph}/IC₅₀ is higher than 30 lasts for 1 hour or more in the lymphatic delivery.

14. The method according to claim 12 or 13, wherein a period of time in which C_{lymph/}IC₅₀ is higher than 30 lasts for 4 hours or less in the lymphatic delivery.

15. The method according to claim 12, wherein blood AUC_{blood} or plasma AUCₚₗₐₛₘₐ as an index of blood exposure of a lipophilic substance which inhibits IL-2 production is 0% to 80% in comparison with that of a control composition.

16. The method according to claim 12, wherein the lipophilic substance which inhibits IL-2 production has a LogP value of 2 or more and an IC₅₀ (a 50% inhibitory drug concentration in a human mixed lymphocyte reaction) of 1 µmol/L or less.

17. The method according to claim 12, wherein the lipophilic substance which inhibits IL-2 production is one or two or more substances selected from the group consisting of tacrolimus, cyclosporin, rapamycin, mycophenolic acid, and mycophenolate mofetil.

18. The method according to claim 12, wherein the carrier is orally administered.

19. The method according to claim 12, wherein the pharmaceutical composition according to any one of claims 1 to 11 is administered.

20. Use of one or two or more substances selected from the group consisting of (i) a mono-, di-, or tri-glyceride, or a mixture thereof, (ii) a fatty acid having 12 to 18 carbon atoms, or a mixture thereof, (iii) a surfactant having an HLB value of 10 to 18 and containing polyethylene glycol having a weight average molecular weight of 200 to 5000 in its molecular structure, and (iv) a higher alcohol having 12 to 18 carbon atoms, as a carrier capable of inhibiting blood exposure of a lipophilic substance which inhibits IL-2 production and delivering the substance to lymph.

21. Use according to claim 20, wherein (i) is one or two or more substances selected from the group consisting of rapeseed oil, castor oil, sunflower oil, cacao butter, medium chain triglycerides, monoolein, diolein, triolein, monoglyceride stearate, diglyceride stearate, triglyceride stearate, monoglyceride palmitate, diglyceride palmitate, triglyceride palmitate, monoglyceride myristate, diglyceride myristate, triglyceride myristate, monoglyceride arachidate, diglyceride arachidate, triglyceride arachidate, monoglyceride behenate, diglyceride behenate, and triglyceride behenate.

22. Use according to claim 20, wherein (ii) is one or two or more substances selected from the group consisting of linoleic acid, stearic acid, oleic acid, lauric acid, and palmitic acid.

23. Use according to claim 20, wherein (iii) is one or two or more substances selected from the group consisting of polyoxyethylene polyoxypropylene glycol, polyoxyethylene fatty acid monoester, saturated polyglycosylated glyceride, D-a-tocopheryl polyethylene glycol 1000 succinate, and polyoxyethylene hydrogenated castor oil.

24. Use according to claim 20, wherein (iv) is one or two or more substances selected from the group consisting of stearyl alcohol, myristyl alcohol, and cetyl alcohol.

25. Use according to claim 20, wherein the lipophilic substance which inhibits IL-2 production has a LogP value of 2 or more and an IC₅₀ (a 50% inhibitory drug concentration in a human mixed lymphocyte reaction) of 1 µmol/L or less.

26. Use according to claim 20, wherein the lipophilic substance which inhibits IL-2 production is one or two or more substances selected from the group consisting of tacrolimus, cyclosporin, rapamycin, mycophenolic acid, and mycophenolate mofetil.
